(19) Europäisches Patentamt
European Patent Office
Office européen des brevets

(11) Publication number: **0 275 716 B1**

(12) **EUROPEAN PATENT SPECIFICATION**

(45) Date of publication of patent specification: **09.08.95**

(51) Int. Cl.[6]: **A61L 15/16**, A61K 31/565

(21) Application number: **87311508.3**

(22) Date of filing: **29.12.87**

(54) **Transdermal estrogen/progestin dosage unit, and fertility control kit comprising said dosage unit.**

(30) Priority: **29.12.86 US 947130**
**16.12.87 US 131462**

(43) Date of publication of application:
**27.07.88 Bulletin 88/30**

(45) Publication of the grant of the patent:
**09.08.95 Bulletin 95/32**

(84) Designated Contracting States:
**AT BE CH DE ES FR GB GR IT LI LU NL SE**

(56) References cited:
**EP-A- 0 196 769**
**US-A- 3 832 252**
**US-A- 4 145 416**
**US-A- 4 624 665**

(73) Proprietor: **RUTGERS, THE STATE UNIVERSITY OF NEW JERSEY**
**Old Oueens Building**
**Somerset and George Streets**
**New Brunswick, NJ 08903 (US)**

(72) Inventor: **Chien, Yie W.,**
**5, West Lake Court,**
**North Brunswick, New Jersey 08902 (US)**
Inventor: **Chien, Te-Yen,**
**82, Nichols Apt., 900, Davidson Road,**
**Piscataway, New Jersey 08854 (US)**
Inventor: **Huang, Yih-Chain,**
**5, Bennington Place,**
**Piscataway, New Jersey 08854 (US)**

(74) Representative: **Watkins, Arnold Jack et al**
**European Patent Attorney**
**Frank B. Dehn & Co.**
**Imperial House**
**15-19 Kingsway**
**London WC2B 6UZ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

The system involves transdermal estrogen/progestin absorption dosage units adapted for adhesion to the female subject desiring fertility control or prevention of an unwanted pregnancy. Additionally, the invention relates to a fertility control kit which utilizes a transdermal system of applying a series of transdermal estrogen/progestin dosage units having a polymer layer adhered to a backing layer and an adhesive layer adhered to the polymer layer, said polymer and adhesive layer having dissolved and/or microdispersed therein estrogen and progestin, respectively, in effective dosage amounts. Preferably, the estrogen used is beta-estradiol or ethinyl estradiol and the progestin used is norethindrone or norgestimate. The dosage units can also be used in estrogenic replacement therapy.

Estrogenic therapies include two main areas, fertility control and estrogenic replacement.

Fertility has been controlled by use of a number of orally administered hormone products. The products are ordinarily a combination of an estrogen and a progestin. A synthetic estrogen is ordinarily used as the estrogen component since the natural estrogen, 17-beta-estradiol, is almost completely destroyed, usually by over 90 percent, when taken orally. It is destroyed to a degree in the digestive tract before it is absorbed but primarily the destructive metabolism of 17-beta-estradiol occurs during the [-]first pass[-] hepatic metabolism. Since such a large amount is destroyed, in order to provide an effective dosage orally, a large excess must be administered with uncertain effectiveness and a large amount of unwanted metabolic products. Therefore, a synthetic estrogen such as ethinyl estradiol normally is orally administered with less than desired results.

The progestin component generally inhibits, as intended, ovulation. Also, in the case of administered progestin, a substantial amount of metabolic breakdown occurs causing undesired metabolic products with undesired effects.

Therefore, in the oral administration of what is commonly referred to as "the pill" or other orally administered products, considerably overdosing is necessary to obtain a high degree of assurance that the desired fertility control will be obtained.

A number of major side effects have reportedly been associated with the administration of oral fertility control preparations, such as thrombophlebitis and thrombosis, pulmonary embolism, coronary thrombosis, myocardial infarction, cerebral thrombosis, cerebral hemorrhage and hypertension. These side effects have been attributed to the estrogen component in the oral preparations. Use of the progestin-only preparations (mini-pill) has been found to eliminate the side effects of estrogen. However, the fertility control is less than that of the combined preparations and the menstrual cycle also becomes more irregular. It has been reported that less incidence of irregular bleeding is observed if the progestin is administered at a more constant rate of delivery. Besides the side effects, the oral fertility control preparations also have the disadvantage of fertility control efficacy depending highly on the degree of patient compliance. The risk of pregnancy is known to increase with each pill missed.

An ideal and patient-acceptable fertility control system should provide the following advantages: minimized side effect, increased ease of administration, rapid termination of treatment, if needed, and improved patient compliance. In recent years, considerable attention has already been directed to the development of implantable, intrauterine, intracervical or intravaginal fertility control delivery systems to provide a prolonged and controlled administration of steroidal hormones to the body for achieving fertility control; however, none of the delivery systems developed so far can be considered as ideal and side effect-free.

Other fertility control means have been used, such as topical creams and intravaginal devices, which deliver combinations of one or more progestins and one or more estrogens, including the naturally-occurring estrogen, 17-beta-estradiol. However, the undesirable aspects of such fertility control systems are evident.

It is, therefore, highly desired that transdermal systems be provided which permit 1) use of the natural estrogen, 17-beta-estradiol,if desired, 2) use of a minimum number of dosage units for each menstrual cycle, such as use of three successive weekly dosage units, and 3) adherence to the skin of the subject which would administer sufficiently high levels of estrogen and progestin hormones to provide high assurance of fertility control without a high amount of undesired metabolic or chemical degradative products. Development of a rate-control transdermal drug delivery system, which is capable of minimizing any individual variability and regional differences in skin permeability, is a necessity to attain a predictable blood level of a drug. The transdermal rate-control drug administration is known to offer several potential advantages for systemic medication: (i) avoidance of the risk and inconvenience of intravenous therapy and of the variability in absorption and metabolism associated with oral therapy; (ii) continuity of drug administration, permitting the use of a pharmacologically-active agent with short biological half-life; (iii)

efficacy can be achieved with lower total daily dosage of drug, because of reduced hepatic first-pass metabolism and continuous drug input; (iv) less chance of over- or under-dosing, as a result of prolonged, programmed delivery of drug at required therapeutic rate; (v) provision of a simplified medication regimen; and (vi) ability to rapidly terminate the drug infusion, if needed, by removal of the drug delivery system from skin surface. Therefore, a transdermal contraceptive delivery system, which is capable of providing on a fast effective basis dual-delivery of an estrogen and a progestin at controlled rates for a specific duration would be an ideal system for achieving fertility regulation in women.

The second main area of estrogenic therapy concerns the need for estradiol replacement therapy. It is caused by menopause (the cessation of ovarian function), oophorectomy (loss of one or both ovaries by surgery) or by pituitary failure. Replacement estrogenic therapy is an important need. Besides the need to alleviate the menopausal symptoms caused by estrogenic steroid deficiency, there are additional contributions of such replacement estrogenic therapy associated with osteoporosis (loss of bone mass) and atherosclerosis. It has been found advantageous to administer also an amount of progestin as a part of such estrogenic replacement therapy. There is clearly a need for improvements in means and methods for estrogenic steroid therapy. Even though it has been found that estradiol itself or estradiol in the form of certain derivatives such as nono-or diesters (e.g., acetate esters) can be absorbed transdermally, it is desired that improved transdermal estradiol and other estrogenic steroid absorption dosage unit forms and processes of transdermal administration be developed.

Provided by this invention is a transdermal fertility control absorption system which permits fertility control by using sequentially three transdermal adhesive dosage units which can easily be applied to a selected skin area.

The first patch ordinarily is applied on the fifth day of a menstrual cycle. The dosage unit is replaced by the second unit after 7 days and the second is replaced by a third at the end of another 7 days. Then, at the beginning of the next menstrual cycle, another sequential course of 3 fertility control patches is again used, which course is repeated again and again as long as desired.

The transdermal estrogen/progestin dosage units of this invention comprise:

a) a backing layer which is impervious to the estrogen and progestin hormones, and which preferably is breathable, especially if the dosage unit is used on a long-term basis, such as for several days;

b) a first layer, which has been applied directly to said backing layer, and which comprises a polymer material having dissolved and/or microdispersed therein a dosage amount of one or more pharmaceutically acceptable estrogen hormones which are absorbable transdermally, preferably 17-beta-estradiol, ethynyl estradiol, or combinations thereof, said polymer material being bioacceptable, compatible with said estrogen hormones and permitting said estrogen hormones to be transmitted therethrough for transdermal absorption, and

c) a second layer which is an adhesive and which has been applied directly to said first layer or optionally to a separating layer which has been applied directly to said first layer, and which is in intimate contact with said first layer, said second layer being made of a material different to the polymer material of the first layer or of a polymer material the same as that of the first layer, and having dissolved and/or microdispersed therein a dosage amount of one or more pharmaceutically acceptable progestin hormones which are absorbable transdermally, said second layer being bioacceptable, compatible with said progestin hormones, and permitting said progestin and estrogen hormones to be transmitted therethrough for transdermal absorption, said second layer comprising a transdermal skin absorption enhancing agent;

said estrogen hormone being stable in said first and second layers and said progestin hormone being stable in said second layer, such that on application of said second layer of said dosage unit to the skin of a subject said estrogen and progestin hormones are transdermally absorbed simultaneously through the skin of the subject; and

said dosage amounts of said estrogen hormones and said progestin hormones being such that said dosage unit is capable of delivering 20-50 mcg/day of 17-beta-estradiol (or an amount of another estrogen having an equivalent estrogenic activity) and 100-1500 mcg/day of norgestimate or norethindrone (or an amount of another progestin having an equivalent progestin activity).

Optionally another layer can be included in the dosage units between the first layer (b) which has present an estrogen and the second layer (c) which has present a progestin. In this separating layer, it is preferable to have present little or no estrogen or progestin. The separating layer can be made of adhesive polymers such as used in making the second layer (c), with a bioacceptable polyisobutylene which permits the estrogen in the first layer (b) to be transmitted for transdermal absorption being presently preferred. Additionally, it is presently preferred that the separating layer be free of any substantial amount of skin absorption enhancing agent.

The estrogen dissolved or microdispersed in the first layer comprises an amount of 17-beta-estradiol or ethinyl estradiol effective in providing the role of estrogen in fertility control or in estrogen replacement and norethindrone or norgestimate dissolved or microdispersed in the second layer comprises an amount which will provide the role of progestin in the desired fertility control system or in estrogen replacement. Desirably, the dosage units will provide the desired rate of transdermal absorption of the estrogen and progestin components for a period of several days, preferably for one week. Use of week-long transdermal dosage units minimize the possibility of missed administration of a dosage in fertility control.

The backing layer is made from materials that are substantially impermeable with regard to the hormones of the transdermal dosage unit. It can be made of polymers such as polyethylene, polypropylene, polyurethane, polyvinylchloride, polyesters such as poly(ethylene phthalate), and foils such as laminates of polymer films with metallic foils such as aluminum foil. If the dosage units are used on a long term basis, such as for a multiple of days, the backing layer preferably has a microporosity to permit passage of sweat and air to minimize any skin hydration.

The first layer is suitably fabricated from biologically acceptable lipophilic or hydrophilic polymers, which will permit the estrogen to be transmitted for transdermal absorption and which provide compatibility and stability for the estrogen. The first layer can preferably be made of a suitable polymeric adhesive, such as a suitable polyacrylic or a silicone adhesive in which the estrogen is stable and microdispersible or soluble. The first layer can also be made using a polymer to fabricate a disc in which the estrogen is microdispersed. The polymer-estrogen mixture is then formed into a layer of an appropriate thickness and suitable surface area and is cured, if desired. The first layer is then adhered to the backing layer. Care must be taken that the polymer selected is compatible with the pharmaceutical, permits its release for transdermal absorption and is free or sufficiently free from any biologically unacceptable components.

Other estrogenic steroid hormones can be used in partial or complete replacement of 17-beta-estradiol, for example, an ester of 17-beta estradiol which is biologically compatible and can be effectively absorbed transdermally and at a rate compatible with the rate of absorption of progestin. Also, it is ordinarily desired that such esters are bioconvertible by components of the skin or other portions of the body, such as hydrolytical enzymes (e.g., esterase), to 17-beta-estradiol. If the derivative is an ester, the derivative can be selected from mono- or di-esters since estradiol has hydroxy groups at the 3- and 17- positions, the 3-mono and 17-mono as well as the 3,17 di-esters can be made by generally known esterification methods. Some ester derivatives will be absorbed more readily than the basic 17-beta-estradiol. In selection of ester derivatives, it is ordinarily preferred that the main estrogen hormone used be absorbed at a rate to provide a desirable amount of the estrogen hormone component on a daily basis in a system which simultaneously effects transdermal absorption of the progestin hormone in an effective daily dosage amount over a several day period, preferably one week.

Regarding the daily dosages of progestin, 100 to 1500 mcg, preferably 125 to 250 mcg/day if the progestin used is norgestimate and 1000 mcg/day is generally satisfactory if the progestin is norethindrone. Regarding estrogen, 25 to 50 mcg/day of estrogen based on 17-beta-estradiol are presently believed desired daily doses for humans.

Finally, the second layer of the dosage unit is assembled with the other layer elements to form the dosage unit. The second layer selected can vary depending on many factors including economic factors such as the type of manufacturing equipment most readily available, the rapidity of absorption desired or other factors. For example, the second layer can be applied directly to the first layer. A skin permeation enhancer compound can be mixed thoroughly with the adhesive polymer which is suitable for adhesion to the skin locus to which the transdermal dosage unit will be applied. The progestin used is also dissolved or microdispersed in the second layer. The second layer can be applied to the first layer by spraying or by solvent casting or laminating. The concentration of skin permeation enhancer compound, if employed, can be reduced in the portion of the second layer coming in contact with the subject to be treated, especially if less than desired adhesion is realized in the second layer, by applying the surface portion of the second layer separately, wherein the adhesive composition has a lower concentration of skin permeation enhancer compound or progestin or both. The second layer is desirably thin in the micron-range thickness, suitably 5-200 microns in thickness, desirably 10 to 180 microns, and preferably 20 to 150 microns in thickness. Also, if desired, an additional adhesive means can be used in the form of a ring adhered to the backing layer which extends beyond the circumference of the first layer.

The optional separating layer if employed is applied to the first layer prior to the assembly of the second layer (c) having present progestin. Alternatively, the separating layer can be applied to the surface of the second layer prior to its assembly into the dosage unit. The separating layer is suitably made of a suitable polyisobutylene.

The absorption rate of one or both of the hormones of the transdermal hormone absorption dosage units of the invention can be increased, such as by having an Enhancing Factor of at least 1.2, preferably at least 1.3, and more preferably at least 1.5 or 2.0. Enhancing Factor is defined as the ratio of normalized permeation rate [in mcg/cm$^2$/hr] of a dosage unit of this invention with skin permeation enhancer/the normalized permeation rate of a corresponding dosage unit without enhancer.

The invention allows the administration of said hormones transdermally by forming hormone-containing dosage units having a first layer which has the estrogen dosage dissolved or dispersed therein, to which first layer is adhered a backing layer, said dosage unit having assembled therewith a second layer, which transports the estrogen and progestin and contains the progestin and transdermal absorption enhancing agent; applying the dosage unit in intimate contact with the skin of the subject treated; and administering the hormones transdermally to said subject to achieve fertility control or estrogen replacement.

In the accompanying drawings:

FIG. 1 is a graph of a series of curves showing cumulative (Q) transdermal absorption of ethinyl estradiol from [-]AD TYPE TDDS[-] (adhesive type transdermal drug delivery system) through hairless mouse skin using seven different formulas.

FIG. 2 is a graph of a series of curves showing cumulative (Q) transdermal absorption of norethindrone from adhesive type transdermal drug delivery system through hairless mouse skin using seven different formulas.

FIG. 3 is a graph showing transdermal absorption rates of ethinyl estradiol across human cadaver skin depending upon transdermal absorption enhancer concentration.

FIG. 4 is a graph showing transdermal absorption rates of norethindrone across human cadaver skin depending upon transdermal absorption enhancer concentration.

FIG. 5 is a graph showing transdermal absorption ratio of ethinyl estradiol through human cadaver skin from dosage units of the invention in which both the polymer layer and adhesive layer are made from adhesive polymer.

The backing layer can be made of any suitable material which is impermeable to the hormones of the polymer layer. The backing layer serves as a protective cover for the first layer and provides also a support function. The backing can be formed so that it is essentially the same size layer as the first layer or it can be of larger dimension so that it can extend beyond the side of the first layer or overlay the side or sides of the first layer and then can extend outwardly in a manner that the surface of the extension of the backing layer can be the base for an adhesive means. For long-term applications, e.g., for seven days, it is desirable to use microporous and/or breathable backing laminates, so hydration or maceration of the skin can be minimized. The adhesive means holds the dosage unit in intimate contact with the skin of the subject treated. Examples of materials suitable for making the backing layer are films of high and low density polyethylene, polypropylene, polyurethane, polyvinylchloride, polyesters such as poly(ethylene phthalate), metal foils, metal foil laminates of such suitable polymer films. Preferably, the materials used for the backing layer are laminates of such polymer films with a metal foil such as aluminum foil. In such laminates, a polymer film of the laminate will usually be in contact with the first layer. The backing layer can be any appropriate thickness which will provide the desired protective and support functions. A suitable thickness will be from 10 to 200 microns. Desirably, the thickness will be from 20 to 150 microns, and preferably be from 30 to 100 microns.

The first layer can also be made from polymer adhesives such as polyacrylic, silicone or other suitable polymer adhesives. The first layer can also be made, for example, from silicone elastomers of the general polydimethylsiloxane structure, such as silicone polymers of the following general formula:

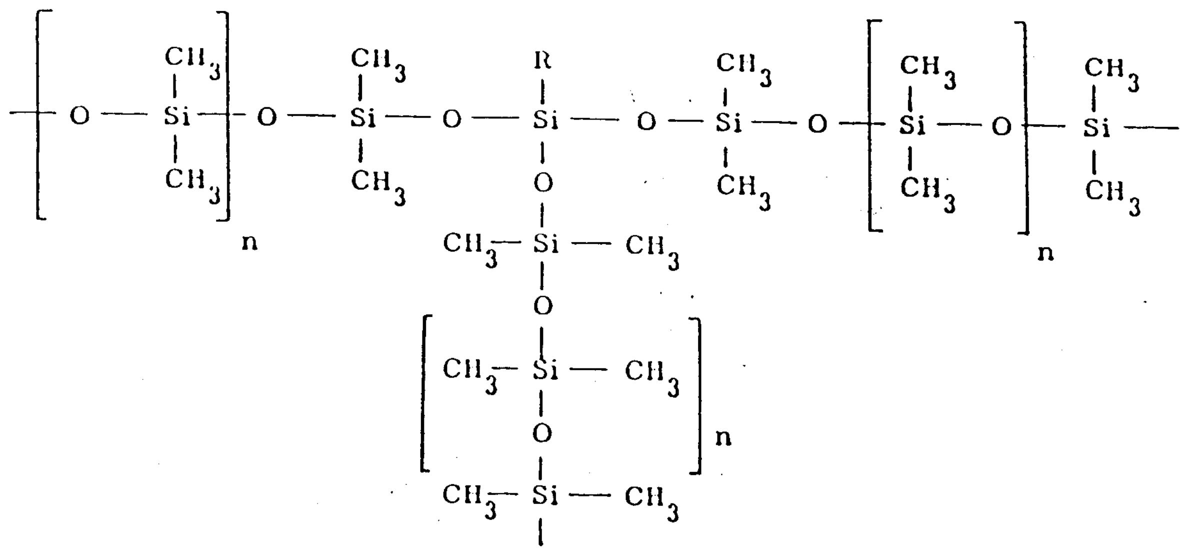

wherein R is alkyl or alkoxy containing 1-7 carbon atoms, vinyl or phenyl and wherein n is 100 to 5000.

The silicone polymers selected preferably are cross-linkable at moderate temperatures, such as room temperature, using cross-linking catalysts which are biologically acceptable in the first layer and which are compatible with the hormone components to be used in making the polymer dosage forms. Various suitable crosslinking agents can be used in crosslinking the above polymer, such as tetrapropoxy silane [$Si(OCH_2CH_2CH_3)_4$], if the silicone polymer has free hydroxy groups such as terminal hydroxy groups. A tin catalyst can be used for such crosslinking reaction. If a silicone polymer component has vinyl groups, it can be crosslinked with a dimethyl-silicone polymer using a catalyst such as a platinum catalyst. Some suitable silicone polymers are cross-linkable copolymers having dimethyl and methylvinyl siloxane units, which can be cross-linked as by using a suitable peroxide catalyst. Other cross-linking sites can be present in the polysiloxane elastomers used. Suitable silicone medical-grade polymers are sold under the designations Silastic 382, Q7-4635, Q7-4650, Q7-4665, Q7-4735, Q7-4750, Q7-4765 and MDX-4-4210.

The silicone polymers selected can also have a "block" or "graft" structure or both. By "block" structure is meant that the polymer can have a section or block of the polymer chain structure of the polymer which can have a repeating unit of one type, such as dimethylsiloxane, and then have a succeeding block made up of repeating units of another type, such as methylvinylsiloxane, diphenylsiloxane, diisopropyl siloxane units or other siloxane or silane units or even of monomer units of a compatible non-siloxane or non-silane type. The blocks can vary in length and be repeated as desired. For example, if the blocks are represented as "A" and "B", respectively, the block copolymer can be A-B or A-B-A or A-B-A-B, etc. The "graft" structure simply means that to the main polymer chain, one or more polymer chains are attached. Those grafted chains can have the same polymer units as those of the main chain or can be different, as described above in connection with "block" copolymers. Also, the polymer used can be of a different type wherein copolymerizable monomers are placed together in a polymerization reactor so the main chain can have a certain population of each of the monomeric units.

The following are examples of block copolymers of the type which can be utilized in this invention.

"A" Block

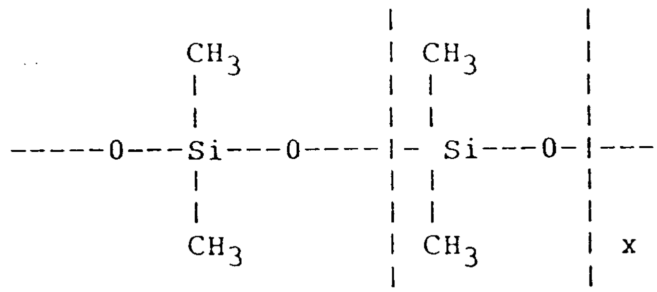

"B" Block

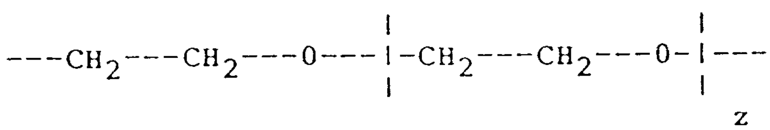

or

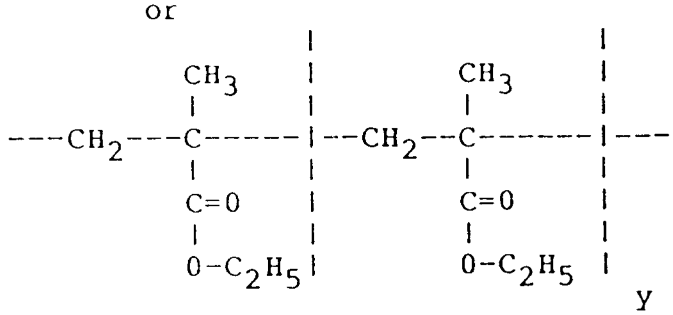

wherein x, y and z represent the number of repeating units sufficient to provide the desired property in the polymer, such as from 10 to 5000.

Generally, those polymers used to form the first layer are those capable of forming thin walls or coatings through which hormones can pass at a controlled rate. Suitable polymers are biologically and pharmaceutically compatible, non-allergenic and insoluble in and compatible with body fluids or tissues with which the device is contacted. The use of soluble polymers is to be avoided since dissolution or erosion of the matrix would affect the release rate of the hormones as well as the capability of the dosage unit to remain in place for convenience of removal.

Exemplary materials for fabricating the first layer include polyethylene, polypropylene, polyurethane, ethylene/propylene copolymers, ethylene/ethylacrylate copolymers, ethylene/vinyl acetate copolymers, silicone elastomers, especially the medical-grade polydimethylsiloxanes, neoprene rubber, polyisobutylene, polyacrylate, chlorinated polyethylene, polyvinyl chloride, vinyl chloride-vinyl acetate copolymer, polymethacrylate polymer (hydrogel), polyvinylidene chloride, poly(ethylene terephthalate), butyl rubber, epichlorohydrin rubbers, ethylene-vinyl alcohol copolymer, ethylene-vinyloxyethanol copolymer; silicone copolymers, for example, polysiloxane-polycarbonate copolymers, polysiloxane-polyethylene oxide copolymers, polysiloxane-polymethacrylate copolymers, polysiloxane-alkylene copolymers (e.g., polysiloxane-ethylene copolymers), polysiloxane-alkylenesilane copolymers (e.g., polysiloxane-ethylenesilane copolymers); cellulose polymers, for example methyl or ethyl cellulose, hydroxypropyl methyl cellulose, and cellulose esters; polycarbonate; polytetrafluoroethylene. For best results, the first layer should be selected

from polymers with glass transition temperatures below room temperature. The polymer may, but need not necessarily, have a degree of crystallinity at room temperature. Cross-linking monomeric units or sites can be incorporated into such polymers. For example, cross-linking monomers can be incorporated into polyacrylate polymers, which provide sites for cross-linking the polymer layer after microdispersing the hormones into the polymer. Known cross-linking monomers for polyacrylate polymers include polymethacrylic esters of polyols such as butylene diacrylate and dimethacrylate, trimethylol propane trimethacrylate. Other monomers which provide such sites include allyl acrylate, allyl methacrylate, diallyl maleate.

The second and first layers are suitably made using a silicone adhesive, such as a (polydimethylsiloxane-silicate resin) copolymer adhesive depicted by the following formula:

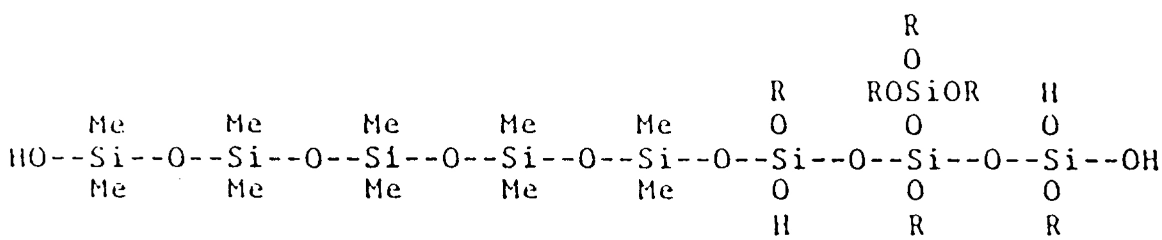

wherein Me is methyl and R is $-Si(CH_3)_3$ and x and y represent independent numbers of repeating units sufficient to provide the desired properties in the second layer and the first layer.

For example, adhesive polymer products or amine-resistant adhesive polymer products sold by Dow Corning, such as the ones sold under the designations of DC-355 and X7-2920 medical adhesives, are suitable for use in making the second layer. The adhesive polymer must be biologically acceptable and compatible with the hormones and skin permeation enhancer, if used. Certain polyacrylic adhesive polymers (in the form of an alkyl ester, amide, free acid, or the like) or polyisobutylene adhesive polymers can also be used with some hormones. Illustrative of suitable adhesive polymers for use in making the first layer are shown by the following formulas:

Polyisobutylene Adhesive

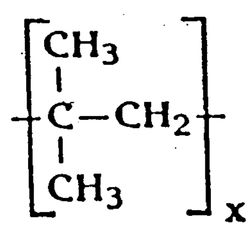

Polyacrylic Adhesive

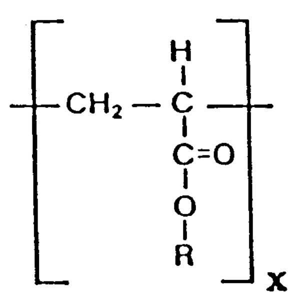

wherein x represents the number of repeating units sufficient to provide the desired properties in the adhesive polymer and R is H or lower alkyl including ethyl, butyl and 2-ethylhexyl.

Other suitable hypoallergenic pressure-sensitive contact adhesive compositions can also be used. A preferred second layer is pressure-sensitive.

However, depending upon pharmaceutical compatibility and other factors, if desired, an additional adhesive means can be in the form of a ring attached, for example, to an extended portion of the backing layer so that the second layer is adjacent to the sidewall of the hormone-containing disc layer. The width of such adjacent adhesive ring must be adequate to hold the dosage unit securely to the subject being treated. Ordinarily, a suitable width of such adhesive ring can be 0.2 to 1.2 cm, preferably 0.3 to 1.0 cm.

The second layer then is finally covered with a releasable protective film layer which is made from materials which are substantially impermeable to the hormones, the skin permeation enhancer, if used, and any other components of the dosage unit. The polymer materials and metal foil laminates used for the backing layer may also be used to make the protective layer, provided the layer is made strippable or releasable such as by applying conventional siliconizing or teflon coating. A suitable releasable material for use with silicone polymer adhesive DC-355 and X7-2970 is Scotchpak 1022 material sold by the 3M Company or Bio-Release Material by Dow Corning.

In making the first layer, silicone elastomers, such as (polydimethylsiloxane-silicate resin) copolymer, polyacrylic adhesive, such as sold under the designation Duro-Tak, of the formula described above, and other biocompatible adhesive polymers which provide a stable environment for the hormones and permit their release, can suitably be used. In making hormone-dispersed dosage units, it has been found suitable to use a dispersing agent, such as lactose and silica. Other suitable dispersing agents can also be used instead so long as they are effective. Other suitable dispersing agents known to the formulating art can be used. Depending upon the hormones and the drug loading desired, a suitable amount of a dispersing agent can be varied from zero to 20 percent (by weight) based on the weight of the first layer. Commonly, the dispersing agent is added together with the hormone into the polymer used in making the layer. The amount of dispersing agent added is dependent upon the rate of permeation desired, the particular hormones used, and at times, other factors. The amount of the hormones added depends upon the amount of hormone dosage and the duration of treatment desired in each dosage unit and the amount which can be incorporated into the polymer layer to retain suitable structural, diffusion and other properties in the first layer. It has been found, for example, that the hormones can be satisfactorily added to 50 parts of the polymer used in making the first layer, such as polyacrylic adhesive polymer. It has been found to be preferable to add and disperse the estrogen used in an amount of a selected adhesive polymer. The mixture of the polymer and hormones is then thoroughly mixed using a high-torque mixer to form a homogeneous solution or microdispersion of the hormones in the polymer. After the mixing step, the composition is subjected to vacuum to remove entrapped air.

The deaereated mixture is then applied as by solvent casting technique, to a suitable substrate, like backing laminate or release liner, and heated to a suitable elevated temperature to remove the solvent. The temperature used should not cause significant degradation of the hormones. The polymer sheet desirably is 10 to 400 microns, preferably 20 to 300 microns, in thickness. The resulting medicated polymer sheet is removed from the casting machine and another layer of medicated polymer, containing the same or different hormone, can be further coated on the first medicated polymer layer formed by direct casting or lamination.

The optional separating layer can be made of the adhesive or other materials described for use in making the second layer having progestin present. In making the separating layer, it has been found suitable to use a bioacceptable polyisobutylene having a suitable molecular weight. For example, the poylisobutylene used can suitably have a relative molecular mass Mv (viscosity average) of from 800,000 to 900,000, such as that of polyisobutylene sold by BASF under the designation Oppanol B80, which has a relative molecular mass Mv (viscosity average) value of 850,000. The viscosity average relative molecular mass is obtained from the equation: $J_o = 3.06 \times 10^{-2} \times M\ 0.65$. The viscosity or molecular weight should, generally speaking, be selected which is sufficiently high to provide a separating layer which is dimensionally stable and which is not excessively high so as to make fabrication of the separating layer unnecessarily difficult to provide a functional and pharmaceutically elegant dosage unit.

The thickness of the separating layer can vary as desired. However, it has been found that a layer thickness after any solvent removal of 50 to 150 microns to be suitable, with a thickness of 75 to 125 microns to be preferable. It has been found that a separating layer having 100 micron thickness made of a polyisobutylene having a viscosity or molecular weight such as that of Oppanol B80 to function well, if the estrogen in the polymer layer is 17-beta-estradiol or ethinyl estradiol.

The separating layer should have sufficient thickness to minimize any migration, especially under prolonged storage conditions at elevated temperatures, such as 37°C or 45°C or greater. Also, the separating layer should be made of a suitable material and with a sufficient thickness to decelerate the rate of transmission of the estrogen in the first layer, as needed to provide suitably a delivery ratio of transdermal absorption from 10-30/1, a ratio of 12 to 20 being a preferable ratio range.

It has been found that the separating layer can be made as by dissolving 10 parts of a suitable polyisobutylene, such as Oppanol B80 polyisobutylene in a suitable solvent, such as a mixture of cyclohexane, hexane and heptane (for example, a 1:1:1 mixture). The mixture is gently agitated such as by using a suitable rotator.

When the dissolution is substantially complete to provide a clear polyisobutylene solution, the solution can be used to coat a low adhesion film, such as a polyester film with a fluoropolymer-coated surface such as the material sold by 3M Company under the designation Scotch-Pak 1022. A R.D. wireless coating bar (such as a #12) can be used for coating. The resulting coating is dried and is repeated as necessary to obtain a layer of desired thickness, such as 100 microns. The separating layer thus formed can be assembled into the dosage unit by lamination to the polymer layer. Alternatively, the separating layer can be applied to the upper surface of the second layer having present progestin before being assembled by lamination to the lower surface of the first layer having present estrogen. The finished multilayered construction can then be cut to form discs with desired shapes and sizes. The disc generally should not exceed 100 sq. cm in area, suitably 5 to 100 sq. cm, preferably, 8 to 80 sq. cm, generally 10 to 60 sq. cm being more preferable. The shape of the discs can vary; they can be circular, square, rectangular or other desired shape.

The first layer, generally speaking, may contain some excess of the dispersed hormone over the dosage amount desired to be transdermally absorbed by the subject to be treated. Ordinarily, this excess is small, such as less than 2-fold excess over a weekly projected dose, depending upon the physiochemical properties of the hormones, as well as the nature of the polymer in the first layer and other factors.

The second layer if it contains a skin permeation enhancer, is made as by dissolving the enhancer and the progestin directly in the adhesive polymer solution or in a solvent which is compatible with the adhesive polymer solution used to make the second layer containing the skin permeation enhancer. Any suitable amount of solvent can be used as necessary to dissolve the quantity of enhancer and progestin to be admixed with the adhesive polymer solution used. For example, 3 to 10 parts of solvent can be used to dissolve one part of skin permeation enhancer, depending upon the solubility of the enhancer. When using polydimethylsiloxane adhesive solution, it has been found suitable to use 2 to 20 parts of skin permeation enhancer in 20 to 50 parts of solvent (such as acetone, methyl ethyl ketone, trifluorotrichloroethane or other suitable solvent) and add the solution to 100 parts of the adhesive solution. The enhancer-adhesive combination is thoroughly mixed and a coating thereof is applied using a film coating machine, such as referred to in the art as a K-bar coater, directly onto the first layer or to a strippable release liner before laminating onto the first layer, as described above. A suitable release liner is a poly(ethylene phthalate) laminated with aluminum foil or a Teflon-coated polyester film such as sold under the designation Scotchpak 1022 or Bio-release X7-2741 or X7-2752. The poly(ethylene phthalate) side to which the second layer coating is applied, is made strippable by conventional siliconizing or by other suitable means. The thickness of the second layer normally is suitable 20 to 200 microns, preferably 30 to 150 microns.

The amount of enhancer in the second layer depends in part on the rapidity at which it is desired that the hormones be absorbed. Generally speaking, 1 to 30 percent of skin permeation enhancer based on the weight of the adhesive is suitable, depending upon the enhancer, adhesive polymer, desired adhesiveness and other factors. Desirably, 5 to 20 percent of skin permeation enhancers are used depending upon the above recited factors. The second layer containing the progestin and skin permeation enhancer is transferred to the first layer surfaces by application of lamination technique under a constant pressure. In order to assure adequate adhesion of the second polymer layer to the skin of the subject treated, additional adhesive polymer coating having a relatively low concentration of enhancer, e.g., 1-5 percent based on the weight of the adhesive polymer can be further applied to the surface of the second layer. The thickness of this coating ordinarily is a minor percentage of the thickness of the final adhesive layer, such as 20-40 percent of the total second layer. In the second layer having a suitable higher concentration of the enhancer is used. Suitable higher concentrations of enhancer are usually 10 to 30 percent based on the adhesive polymer weight, the solubility and desired final amount of skin enhancer agent and other factors. The solvent of the respective coatings is removed by evaporation. The respective coatings can be combined to make the final multilayered fertility-control transdermal dosage unit by application of lamination technique under a constant pressure or sequential solvent casting technique.

The multi-layer transdermal hormone dosage units are excised. The backing layer, if desired, can be shaped around the sides of the dosage unit, including the first layer, if such protection is desired. The resulting dosage units are then placed in appropriate packaging for storage until they are to be applied in transdermal treatment.

At least one estrogen and at least one progestin as defined above are dissolved and/or microdispersed in the first and second layers, respectively. With the controlled release of the hormones at a relatively steady rate over a prolonged period, typically several days and preferably one week, the subject is provided with the benefit of a steady infusion of the hormones over a prolonged period.

One of the presently preferred estrogens is 17-beta-estradiol. It is a natural hormone and ordinarily transdermally delivered by an adaptable system of this invention at a desirable daily rate. The 17-beta-estradiol is compatible and can be dissolved or microdispersed in the polymer. The transdermal dosage unit designed for one-week therapy is required to deliver at least 100 to about 500 mcg (preferably 125 to 250 mcg)/day of norgestimate or about 1000 mcg/day of norethindrone and 20-50 mcg/day of 17-beta-estradiol (or an equivalent effective amount of ethinyl estradiol or another estrogen). In fertility control, that amount of progestin is believed to be necessary to inhibit ovulation and that amount of estrogen is believed needed to maintain normal female physiology and characteristics. Derivatives of 17-beta-estradiol which are biocompatible, capable of being absorbed transdermally and preferably bioconvertible to 17-beta-estradiol can also be used, if the amount of absorption meets the required daily dose of the estrogen component and if the hormone components are compatible. Such derivatives of estradiol can be selected from esters, either mono- or di-esters. The monoesters can be either 3- or 17- esters. The estradiol esters can be, illustratively speaking, estradiol-3,17-diacetate; estradiol-3-acetate; estradiol-17-acetate; estradiol-3,17-divalerate; estradiol-3-valerate; estradiol-17-valerate; 3-mono, 17-mono and 3,17-dipivilate esters; 3-mono, 17-mono and 3,17-dipropionate esters; corresponding cypionate, heptanoate, and benzoate; ethinyl estradiol; estrone; and other estrogenic steroids and derivatives thereof which are transdermally absorbable, including benzestrol, chlorotrianisene, dienestrol, and mestranol.

The progestin can be and presently preferably is norethindrone or norgestimate or combinations thereof. However, other suitable progestins can be used in place thereof or in combination therewith. For example, a progestin can be selected from levonorgestrel, norethynodrel, dydrogesterone, ethynodiol acetate, hydroxyprogesterone caproate, medroxyprogesterone diacetate, norethindrone acetate, norgestrel, and progesterone.

It will be suggested to those skilled in the art to use other estrogens or progestins in forming the dosage units of this invention. Such use of other estrogens and progestins are intended to be within the scope of this invention insofar as use thereof provides satisfactory dosage units within the spirit of this invention.

The skin permeation enhancers which can be used in carrying out this invention can vary. Ones that give preferred results with the polymer dosage unit form having a specific hormone can vary. In some instances, the use of permeation enhancer in making a dosage unit will result in good or even excellent absorption for one hormone, may result in no or relatively low enhancement when another hormone is used. Use of combinations of two or more of the skin permeation enhancer compounds frequently result in superior results, such as greater transdermal absorption.

Specific skin permeation enhancers which can be used in making the polymer dosage forms of this invention include saturated and unsaturated fatty acids and their esters, alcohols, monoglycerides, acetate, diethanolamides and N, N-dimethylamides, such as oleic acid, propyl oleate, oleyl acetate, propyl myristate, isopropyl myristate, myristyl alcohol, myristyl N, N-dimethyl amide, stearic acid and stearyl alcohol, stearyl propyl ester, monostearin, and combinations of them with, for example, 1-dodecylazacycloheptan-2-one sold under the trademark Azone by Nelson Research and Development; decyl methyl sulfoxide, dimethyl sulfoxide, salicylic acid and derivatives, N,N-diethyl-m-toluamide, crotamiton, 1-substituted azacycloalkan-2-ones such as disclosed in U.S. Patent 4,316,893 (the 1-substituent having 0-17 carbon atoms, preferably, 1-11 carbon atoms), and various other compounds which are biologically compatible and have transdermal permeation enhancement activity. It has been found that n-decyl alcohol is a preferred enhancing agent. Also, it has been found that capric acid is a preferred enhancing agent. Modifications will be suggested to those skilled in the art to the chemical structures represented by n-decyl alcohol or capric acid which do not detract substantially from their function as preferred enhancing agent. It has been found that 10 to 40 percent (W/W) of n-decyl alcohol or capric acid is ordinarily a suitable amount. It has been found that 20 to 35 percent (W/W) in the second layer of these enhancing agents provide highly satisfactory skin absorption enhancement and satisfactory adhesion. Amounts higher than 35 or 40 percent (W/W) can diminish skin absorption enhancement and interfere with satisfactory adhesion to the subject being treated.

Ethyl alcohol and other short chain alkanols (with 1-4 carbon atoms) which have substantially the same properties and activity as ethyl alcohol do not come within the definition of skin permeation enhancer as used herein.

The following examples are in illustration of the invention and are not intended to be limiting.

## Example 1

The following ingredients are used in making the first layer: ethinyl estradiol, 5 parts; polyacrylic adhesive sold by National Starch and Chemical Corp. as Duro-Tak 80-1054, 100 parts.

The ethinyl estradiol is added to the polyacrylic adhesive using a high torque mixer (sold by Cole-Parmer Company) at a rate of about 1000 RPM.

The hormone mixture is applied to the backing layer formed of polyester/aluminum laminate sold by 3M Company as Scotch-Pak 1005, by using a K-bar coater equipped with a number 3 bar. The resulting first layer is dried in the hood for one hour to remove the solvents. The thickness of the first layer obtained is about 10 microns.

To the first layer, a 5% (W/W) norethindrone in polyacrylic adhesive solution is applied using a K-bar coater (with #16 bar). In addition to norethindrone, this coating solution also contains 25% (W/W) of n-decyl alcohol as skin permeation enhancer. The coating is dried at ambient room temperature for 24 hours. The dried norethindrone-reservoir second layer has a dry thickness of about 120 microns.

The bilayer dosage units are then covered with a transparent low-adhesion release liner (Scotch-Pak 1022/3M). The completed dosage layers are then cut into dosage units having various shapes and sizes by using a specially-designed device cutter, such as a 20 $cm^2$ rectangular shape.

The transdermal absorption of the hormones from the anti-fertility polymer dosage units of this invention is evaluated by using a skin specimen from a "hairless" mouse or human cadaver by following the procedure described by Y.W. Chien, K. Valia and U.B. Doshi in Drug Develop. & Ind. Pharm., 11(7) 1195-1212 (1985).

Transdermal polymer dosage units are obtained following generally the procedures described above and evaluated as shown in the following Tables 1-3.

TABLE 1

| Rates of Permeation[1)] for Ethinyl Estradiol and Norethindrone Across Hairless Mouse Skin[2)] | | | |
|---|---|---|---|
| Formulation | Enhancer | Permeation Rate ($mcg/cm^2/hr \pm$ S.D.) (n = 3) | |
| | | Ethinyl Estradiol[3)] | Norethindrone[4)] |
| 1 | none | 0.04 (± 0.01) | 0.45 (± 0.07) |
| 2 | IPM | 0.25 (± 0.05) | 0.97 (± 0.19) |
| 3 | DMSO | 0.13 (± 0.02) | 0.52 (± 0.14) |
| 4 | DeMS | 0.16 (± 0.03) | 0.39 (± 0.07) |
| 5 | LA | 0.19 (± 0.03) | 0.76 (± 0.20) |
| 6 | OA | 0.26 (± 0.04) | 1.21 (± 0.22) |
| 7 | DeA | 0.30 (± 0.06) | 2.51 (± 0.49) |
| 8 | CA | 0.25 (± 0.04) | 2.04 (± 0.30) |

[1)] 12 samples were taken during 115 hours of study.
[2)] Seven-week-old female hairless mouse abdominal skin.
[3)] Loading dose: 30.5 (±1.3) $mcg/cm^2$
[4)] Loading dose: 338.0 (±10.1) $mcg/cm^2$
IPM (isopropyl myristate); DMSO (dimethyl sulfoxide); DeMS (decyl methyl sulfoxide); LA (lauric acid); OA (oleic acid);

TABLE 2

| Rates of Permeation[1)] for Ethinyl Estradiol and Norethindrone Across Human Cadaver Skin[2)] | | | |
|---|---|---|---|
| Formulation | Enhancer | Permeation Rate ($mcg/cm^2/hr$ ± S.D.) (n = 3) | |
| | | Ethinyl Estradiol[3)] | Norethindrone[4)] |
| 1 | none | 0.02 (± 0.004) | 0.14 (± 0.03) |
| 2 | IPM | 0.09 (± 0.02) | 0.39 (± 0.07) |
| 3 | DMSO | 0.04 (± 0.01) | 0.17 (± 0.03) |
| 4 | DeMS | 0.05 (± 0.01) | 0.12 (± 0.02) |
| 5 | LA | 0.07 (± 0.02) | 0.26 (± 0.04) |
| 6 | OA | 0.09 (± 0.02) | 0.46 (± 0.09) |
| 7 | DeA | 0.13 (± 0.02) | 0.89 (± 0.18) |
| 8 | CA | 0.07 (± 0.01) | 0.80 (± 0.14) |

1) 12 samples were taken during 122 hours of study.
2) A 17-year-old black boy's left anterial leg with average thickness of 220 (± 26) microns.
3) Loading dose: 30.5 (±1.3) $mcg/cm^2$.
4) Loading dose: 338.0 (±10.1) $mcg/cm^2$.

TABLE 3

| Enhancing Effect of Skin Permeation Enhancer on the Skin Permeation Rates[1)] of Ethinyl Estradiol and Norethindrone Across Human Cadaver Skin[2)] | | | |
|---|---|---|---|
| Formulation | Enhancer | Permeation Rate ($mcg/cm^2/hr$ ± S.D.) (n = 3) | |
| | | Ethinyl Estradiol[3)] | Norethindrone[4)] |
| | n-Decyl Alcohol (% w/w) | | |
| 9 | 0 | 0.13 (± 0.02) | 0.10 (± 0.02) |
| 10 | 2.5 | 0.15 (± 0.03) | 0.09 (± 0.02) |
| 11 | 5.0 | 0.36 (± 0.06) | 0.17 (± 0.03) |
| 12 | 10.0 | 0.48 (± 0.07) | 0.25 (± 0.04) |
| 13 | 25.0 | 1.28 (± 0.19) | 1.24 (± 0.18) |
| 14 | 50.0 | 1.06 (± 0.18) | 1.45 (± 0.21) |
| | Capric acid (% w/w) | | |
| 15 | 1.0 | 0.12 (± 0.02) | 0.08 (± 0.01) |
| 16 | 2.5 | 0.13 (± 0.02) | 0.08 (± 0.01) |
| 17 | 5.0 | 0.19 (± 0.03) | 0.13 (± 0.02) |
| 18 | 10.0 | 0.26 (± 0.04) | 0.23 (± 0.04) |
| 19 | 25.0 | 0.39 (± 0.07) | 0.33 (± 0.05) |
| 20 | 50.0 | 0.40 (± 0.06) | 0.49 (± 0.10) |

1) 13 samples were taken during 168 hours of study period.
2) A white male's anterial trunk with average thickness of 180 ± 20 microns (n = 6) were used.
3) Loading dose: 95.7 (± 2.9) $mcg/cm^2$
4) Loading dose: 1.26 (± 0.03) $mg/cm^2$

Example 2

The formulations of the above Tables are repeated using the following procedure.

The following ingredients are used in making the first layer: ethinyl estradiol, 5 parts; polyacrylic adhesive sold by National Starch and Chemical Corp. as Duro-Tak 80-1054, 100 parts.

The ethinyl estradiol is added to the polyacrylic adhesive using a high torque mixer (sold by Cole-Parker Company) at a rate of about 1000 RPM.

The hormone mixture is applied to the backing layer formed of polyester/aluminum laminate sold by 3M Company as Scotch-Pak 1005, by using a K-bar counter equipped with a number 3 bar. The resulting first layer is dried in the hood for one hour to remove the solvents. The thickness of the first layer obtained is about 10 microns.

To the first layer, a 5% (W/W) norethindrone in polyacrylic adhesive solution is applied on a transparent low-adhesion substrate using a K-bar coater (with #16 bar). In addition to norethindrone, this coating solution also contains 25% (W/W) of n-decyl alcohol as skin permeation enhancer. The coating is dried in the hood at ambient room temperature for 24 hours. The dried norethindrone-reservoir second layer has a thickness of about 120 microns.

The norethindrone layer is carefully applied to the ethinyl estradiol layer by lamination technique. The completed dosage layers are then cut into dosage units having various shapes and sizes by using a specially-designed device cutter, such as 20 $cm^2$ rectangular shape.

Example 3

Example 1 is repeated except the ethinyl estradiol loading in the first layer is varied from 200 mcg/20 $cm^2$ to 1600 mcg/20 $cm^2$. The above formulation 13 is used. The data show that the rate of permeation across human skin increases as the loading of ethinyl estradiol increases until the loading concentration reaches about 1600 mcg/20 $cm^2$, at which point increased loadings according to the data of the experiment do not provide increased permeability. This is shown in the chart of FIG. 5.

Example 4

Examples 1 and 2 are repeated except bioactively equivalent amounts of 17-beta-estradiol are used instead of ethinyl estradiol.

Example 5

Examples 1 and 2 are repeated except bioactively equivalent amounts of norgestimate are used instead of norethindrone.

Example 6

Examples 1 and 2 are repeated except bioactively equivalent amounts of 17-beta-estradiol and norgestimate are used instead of ethinyl estradiol and norethindrone, respectively.

Example 7

Examples 1, 2, 4, 5 and 6 are repeated using polydimethylsiloxane adhesive instead of the polyacrylic adhesive.

Example 8

The following ingredients are used in making a tri-layer transdermal estrogen/progestin dosage unit:

(I) Ethinyl estradiol, 1 part; polyacrylic adhesive (sold by National Starch and Chemical Corp., as Duro-Tak 80-1054), 99 parts. The ethinyl estradiol is dissolved in the polyacrylic adhesive by gently rotating the container using rotator (Cole-Parmer Company) at low speed (10 rpm) to form a clear solution. This hormone/adhesive mixture is applied by coating to a sheet of polyester/aluminum laminate (sold by 3M Company as Scotch-Pak 1109), on the polyester surface, using a R.D. wireless-coating bar (#8). The resulting first layer is dried in the hood for one hour to remove the solvent portion. The thickness of the dried first layer obtained is about 40 microns.

(II) Polyisobutylene polymer (sold by BASF, as Oppanol B80), 10 parts; 1:1:1 mixture of cyclohexane/hexane/heptane as solvent system for Oppanol B80, 90 parts. The polyisobutylene polymer is dissolved in the solvent system in a closed container by gently rotating the container using a rotator (Cole-Palmer Company) at low speed (10 rpm) until all the polymer is dissolved and a clear solution is formed. This polymer solution is applied to the low-adhesion side of a substrate (a polyester film with fluoropolymer-coated surface, sold by 3M Company as Scotch-Pak 1022) using R.D. wireless coating bar (#12). This polymer coating is dried in the hood for 2 hours. The resulting dried polymer film has a thickness of about 100 microns.

(III) Norethindrone, 5 parts; n-decyl alcohol, 35 parts; polyacrylic adhesive (sold by National Starch and Chemical Corp. as Duro-Tak 80-1054), 60 parts. The norethindrone is dispersed in n-decyl alcohol by rotating gently the container using a rotator (Cole-Palmer Company) at low speed (10 rpm) to form a drug suspension. The polyacrylic adhesive is then added to the suspension and the mixture is rotated gently again using the same rotator at a speed of 10 rpm until a homogeneous mixture is obtained. The mixture is applied to the low-adhesion side of a substrate (a polyester with fluoropolymer-coated surface, sold by 3M Company as Scotch Pak 1022) using a R.D. wireless coating bar (#28). This coating layer is dried in the hood for 24 hours. The thickness of the dried norethindrone/n-decyl alcohol reservoir layer obtained is about 250 microns.

The polyisobutylene product of (II) is laminated onto the product of (I) containing ethinyl estradiol. The layer of (III) containing norethindrone is then laminated into the combined laminates (I) and (II), on the surface of layer (II), to form the final product. The final laminated product is cut into specific size using steel die cutter to form the tri-layer transdermal estrogen/progestin dosage unit.

Units of 10 sq cm are individually packed in the paper/foil/polyester pouches which are then thermally sealed by a thermal sealer. These sealed pouches are stored in the stability testing cabinets (Gravity Convection Incubator, sold by GCA Corp.) at three different temperatures, room temperature, 37°C and 45°C for up to 26 weeks. During the storage, pouches are randomly sampled at specific intervals, according to the sampling schedule shown in Table 4. The units sealed in the pouches are evaluated for their drug content and skin permeation rate profiles. Drug content in each unit was determined by a solvent extraction procedure followed by a high performance liquid chromatograph (HPLC) of the drugs. The skin permeation rate profiles of drugs released from the unit were determined by a hydrodynamically well-calibrated in-vitro skin permeation cell system. The skin specimen freshly excised from 5-to-7 week old female hairless mouse skin was used as the model skin. The skin permeation study was performed at 37°C using 40% V/V PEG 400 saline solution as receptor solution. The steady-state skin permeation rate of norethindrone and ethinyl estradiol was determined from the slope of a Q vs time plot, where Q is the cumulative amount of drug permeating through the skin at a specific sampling time interval. It was calculated by determining the drug concentration in the receptor solution by the HPLC assay.

Drug content or skin permeation rate of drug determined from the stability samples were plotted, according to storage temperature, against the storage time. A 95% confidence limit, based on the mean value obtained from the week 0 samples, is established to make statistical judgment on the physical and chemical stability of the unit tested. Data point that falls outside the 95% confidence limit lines is considered as either chemically (from drug recovery study) or physically (from skin permeation study) unstable.

TABLE 4

| Temperature (°C) | Sampling Schedule (Weeks) | | | | | | |
|---|---|---|---|---|---|---|---|
| | 0 | 1 | 2 | 4 | 8 | 12 | 26 |
| Room | X | - | - | X | X | X | X |
| 37 | X | - | X | X | X | X | X |
| 45 | X | X | X | X | X | X | X |

TABLE 5

Drug Recovery Data of Stability Samples of Dosage Units of Example 8

| Temperature | 0 | 1 | 2 | 4 | 8 | 12 | 26 |
|---|---|---|---|---|---|---|---|
| | Sampling Time (Weeks After Storage) | | | | | | |
| | Ethinyl Estradiol (mcg/10 $cm^2$ ± S.D.)[1,2] | | | | | | |
| Room Temp. | 529.7 (32.98) | --- | --- | 514.5 (35.57) | 527.7 (34.99) | 517.6 (27.89) | 511.1 (31.31) |
| 37°C | 552.2 (33.89) | --- | 576.8 (37.22) | 539.5 (36.67) | 531.1 (29.45) | 538.9 (43.41) | 522.1 (29.82) |
| 45°C | 540.4 (33.71) | 530.7 (39.98) | 522.7 (37.11) | 539.6 (29.67) | 513.6 (29.98) | 505.7 (42.11) | 495.2 (34.72) |
| | Norethindrone (mcg/10 $cm^2$ ± S.D.)[1,2] | | | | | | |
| Room Temp. | 14.18 (0.98) | --- | --- | 13.43 (1.06) | 13.11 (1.18) | 14.14 (1.32) | 13.01 (1.24) |
| 37°C | 16.67 (1.09) | --- | 15.57 (1.41) | 15.11 (1.11) | 14.40 (1.61) | 14.11 (1.24) | 13.79 (1.18) |
| 45°C | 15.22 (1.43) | 15.01 (1.14) | 14.77 (1.02) | 14.78 (1.31) | 13.49 (1.29) | 13.29 (1.08) | 13.77 (1.19) |

1) Mean ± Standard Deviation (N = 3)

2) Triplicate sample dosage units solvent extracted at indicated storage times and temperatures and the ethinyl estradiol and norethindrone contents determined using high performance liquid chromatography (HPLC).

TABLE 6

Skin Permeation Rate of Stability Samples of Dosage Units of Example 8

| Temperature | Sampling Time (Weeks After Storage) 0 | 1 | 2 | 4 | 8 | 12 | 26 |
|---|---|---|---|---|---|---|---|
| | Ethinyl Estradiol ($mcg/10\ cm^2$ hr ± S.D.)[1,2] | | | | | | |
| Room Temp. | 0.20 (0.034) | --- | --- | 0.26 (0.033) | 0.19 (0.021) | 0.26 (0.033) | 0.22 (0.021) |
| 37°C | 0.25 (0.027) | --- | 0.27 (0.040) | 0.22 (0.018) | 0.29 (0.027) | 0.30 (0.041) | 0.22 (0.031) |
| 45°C | 0.28 (0.041) | 0.25 (0.022) | 0.21 (0.026) | 0.31 (0.039) | 0.22 (0.033) | 0.23 (0.042) | 0.20 (0.031) |
| | Norethindrone ($mcg/10\ cm^2$ hr ± S.D.)[1,2] | | | | | | |
| Room Temp. | 2.46 (0.26) | --- | --- | 2.38 (0.31) | 2.09 (0.19) | 2.15 (0.25) | 2.57 (0.35) |
| 37°C | 2.33 (0.31) | --- | 2.46 (0.41) | 2.37 (0.35) | 2.72 (0.41) | 2.44 (0.39) | 2.21 (0.34) |
| 45°C | 2.29 (0.33) | 2.56 (0.37) | 2.33 (0.28) | 2.71 (0.37) | 2.53 (0.37) | 2.56 (0.35) | 2.28 (0.31) |

1) Mean ± Standard Deviation (N = 3)

2) Permeation rates of triplicate sample dosage units determined using 5-7 week old female hairless mouse skin in Chien et al. procedure for 146 hours and the rates determined from slope of Q vs permeaticn time plots.

TABLE 7

| Effect of Thickness of Polyisobutylene Layer (Oppanol B80) on Permeation Rates Across Human Cadaver Skin | | |
|---|---|---|
| Formulation Number | Human Cadaver Skin Permeation Rates[a,b,c,d] (mcg/sq cm hr ± S.D.) | |
| | Ethinyl Estradiol | Norethindrone |
| 2-0 | 0.22 (0.041) | 1.15 (0.100) |
| 2-1 | 0.24 (0.033) | 1.03 (0.170) |
| 2-2 | 0.20 (0.021) | 1.24 (0.210) |
| 2-3 | 0.16 (0.029) | 0.97 (0.160) |
| 2-4 | 0.16 (0.031) | 1.19 (0.240) |
| 2-5 | 0.14 (0.021) | 1.04 (0.250) |
| 2-6 | 0.09 (0.017) | 1.26 (0.280) |
| 2-7 | 0.06 (0.014) | 1.06 (0.190) |
| 2-8 | 0.04 (0.010) | 1.17 (0.200) |
| 2-9 | 0.02 (0.006) | 1.06 (0.230) |

a. 11 samples were taken for each of the triplicate experiments (n = 3) during 146 hours of study.
b. 40% PEG 400/saline was used as receptor solution.
c. Anterior trunk of a young caucasian female cadaver skin was used.
d. Procedure of Chien et al. used, samples taken at times 0, 2, 4, 8, 12, 24, 48, 72, 96, 120 and 146, and rates determined from slopes of Q vs permeation time plots.

TABLE 8

| Variation of Permeation Rates of Norethindrone Depending on Content of Enhancer | | |
|---|---|---|
| Formulation Number | % (W/W) of n-Decyl Alcohol | Norethindrone Skin Permeation Rate[1,2] (mcg/sq cm hr ± S.D.) |
| 2-10 | 0 | 0.13 (± 0.02) |
| 2-11 | 10 | 0.31 (± 0.07) |
| 2-12 | 20 | 0.49 (± 0.06) |
| 2-13 | 30 | 0.87 (± 0.17) |
| 2-7 | 35 | 1.10 (± 0.24) |
| 2-14 | 40 | 1.17 (± 0.21) |
| 2-15 | 45 | 1.23 (± 0.29) |

1) 11 samples were taken for each of triplicate experiments (n = 3) during 146 hours of study.
2) Procedure of Chien et al. used, samples taken at times 0, 2, 4, 8, 12, 24, 48, 72, 96, 120 and 146, and rates determined from slopes of Q vs permeation time plots.

TABLE 9

| Variation of Permeation Rate Ratios Depending on Enhancer Contents | | |
|---|---|---|
| Formulation Number | % (W/W) of n-Decyl Alcohol | Ratio of Permeation Rates Norethindrone/Ethinyl Estradiol |
| 2-10 | 0 | 4.19 (± 0.41) |
| 2-11 | 10 | 8.38 (± 0.77) |
| 2-12 | 20 | 12.56 (± 1.49) |
| 2-13 | 30 | 18.13 (± 2.33) |
| 2-7 | 35 | 18.33 (± 2.74) |
| 2-14 | 40 | 15.39 (± 1.96) |
| 2-15 | 45 | 13.98 (± 1.87) |

TABLE 10

| Variation of Permeation Rates of Norethindrone Depending on Content of Enhancer | | |
|---|---|---|
| Formulation Number | % (W/W) of Capric Acid | Norethindrone Skin Permeation Rate[1,2] (mcg/sq cm hr ± S.D.) |
| 2-10 | 0 | 0.13 (± 0.02) |
| 2-16 | 10 | 0.41 (± 0.07) |
| 2-17 | 20 | 0.66 (± 0.11) |
| 2-18 | 30 | 1.16 (± 0.19) |
| 2-19 | 35 | 1.48 (± 0.24) |
| 2-20 | 40 | 1.59 (± 0.34) |
| 2-21 | 45 | 1.84 (± 0.15) |

1) 11 samples were taken for each of triplicate experiments. (n = 3) during 146 hours of study.
2) Procedure of Chien et al. used, samples taken at times 0, 2, 4, 8, 12, 24, 48, 72, 96, 120 and 146, and rates determined from slopes of Q vs permeation time plots.

TABLE 11

| Variation of Permeation Ratios Depending on Enhancer Contents | | |
|---|---|---|
| Formulation Number | % (W/W) of Capric Acid | Ratio of Permeation Rates Norethindrone/Ethinyl Estradiol |
| 2-10 | 0 | 4.19 (± 0.41) |
| 2-16 | 10 | 12.42 (± 1.96) |
| 2-17 | 20 | 14.04 (± 2.11) |
| 2-18 | 30 | 15.68 (± 1.91) |
| 2-19 | 35 | 15.91 (± 1.77) |
| 2-20 | 40 | 12.82 (± 2.01) |
| 2-21 | 45 | 12.35 (± 1.44) |

TABLE 12

| Variation of Permeation Rates of Ethinyl Estradiol Depending on Thickness of Adhesive Layer | | |
|---|---|---|
| Formulation Number | Thickness of Adhesive Layer (Microns) | Ethinyl Estradiol Skin Permeation Rate[1,2] (mcg/sq cm hr ± S.D.) |
| 2-22 | 142.8 | 0.079 (± 0.009) |
| 2-23 | 178.5 | 0.070 (± 0.010) |
| 2-24 | 214.2 | 0.066 (± 0.008) |
| 2-7 | 250.0 | 0.060 (± 0.011) |
| 2-25 | 285.6 | 0.066 (± 0.015) |
| 2-26 | 321.7 | 0.074 (± 0.011) |

1) 11 samples were taken for each of triplicate experiments. (n = 3) during 146 hours of study.
2) Procedure of Chien et al.used, samples taken at times 0, 2, 4, 8, 12, 24, 48, 72, 96, 120 and 146, and rates determined from slopes of Q vs permeation time plots.

TABLE 13

| Variation of Ratios of Permeation Rates Depending on Thickness of Adhesive Layer | | |
|---|---|---|
| Formulation Number | Thickness of Adhesive Layer (Microns) | Ratio of Permeation Rates Norethindrone/Ethinyl Estradiol |
| 2-22 | 142.8 | 3.92 (± 0.52) |
| 2-23 | 178.5 | 8.00 (± 1.33) |
| 2-24 | 214.2 | 12.27 (± 2.02) |
| 2-7 | 250.0 | 18.33 (± 2.34) |
| 2-25 | 285.6 | 18.03 (± 2.17) |
| 2-26 | 321.7 | 17.16 (± 1.99) |

The following Tables 14 and 15 show data for dosage units without separating layers in comparison with data shown in Tables 5 and 6 for dosage units with separating layers.

TABLE 14

Drug Recovery Data from Stability Samples of Bi-Layer Patch System[2)]

| Temperature | Sampling Time (Weeks After Storage) 0 | 1 | 2 | 4 | 8 | 12 | 26 |
|---|---|---|---|---|---|---|---|
| | Ethinyl Estradiol (mcg/10 $cm^2$ ± S.D.)[1)] | | | | | | |
| Room Temp. | 566.9 (33.18) | --- | --- | 569.4 (34.44) | 536.8 (33.90) | 552.2 (33.11) | 539.9 (35.56) |
| 37°C | 572.5 (40.26) | --- | 570.0 (34.67) | 545.1 (41.11) | 557.5 (31.22) | 539.9 (33.31) | 533.6 (29.98) |
| 45°C | 559.4 (38.88) | 566.9 (44.76) | 530.5 (39.77) | 527.7 (41.12) | 510.0 (44.17) | 519.8 (36.66) | 507.6 (38.98) |
| | Norethindrone (mcg/10 $cm^2$ ± S.D.)[1)] | | | | | | |
| Room Temp. | 15.52 (0.99) | --- | --- | 14.98 (0.92) | 14.16 (0.79) | 15.78 (1.05) | 16.12 (1.11) |
| 37°C | 16.11 (1.19) | --- | 16.64 (0.84) | 15.52 (0.91) | 14.22 (0.77) | 15.78 (1.17) | 14.02 (0.69) |
| 45°C | 17.72 (1.27) | 17.01 (1.00) | 15.24 (0.92) | 14.96 (1.33) | 14.66 (1.07) | 15.28 (1.26) | 14.06 (0.93) |

1) Mean ± Standard Deviation (N = 3).

2) Triplicate sample dosage units solvent extracted at indicated storage times and temperatures and the ethinyl estradiol and norethindrone contents determined using high performance liquid chromatography (HPLC).

**TABLE 15**

**Skin Permeation Rate from Stability Samples of Bi-Layer Patch System [2)]**

**Sampling Time (Weeks After Storage)**

| Temperature | 0 | 1 | 2 | 4 | 8 | 12 | 26 |
|---|---|---|---|---|---|---|---|
| | Ethinyl Estradiol (mcg/10 $cm^2$ hr ± S.D.)[1)] | | | | | | |
| Room Temp. | 0.59 (0.04) | --- | --- | 0.52 (0.06) | 0.55 (0.05) | 0.59 (0.04) | 0.64 (0.08) |
| 37°C | 0.61 (0.08) | --- | 0.63 (0.09) | 0.70 (0.07) | 0.73 (0.11) | 0.66 (0.10) | 0.79 (0.14) |
| 45°C | 0.66 (0.11) | 0.69 (0.13) | 0.76 (0.13) | 0.77 (0.08) | 0.74 (0.09) | 0.77 (0.16) | 0.87 (0.12) |
| | Norethindrone (mcg/10 $cm^2$ hr ± S.D.)[1)] | | | | | | |
| Room Temp. | 2.51 (0.21) | --- | --- | 2.58 (0.26) | 2.44 (0.31) | 2.31 (0.13) | 2.20 (0.23) |
| 37°C | 2.88 (0.21) | --- | 2.95 (0.20) | 2.39 (0.15) | 2.33 (0.17) | 2.22 (0.12) | 2.07 (0.11) |
| 45°C | 2.74 (0.33) | 2.61 (0.22) | 2.50 (0.16) | 2.31 (0.15) | 1.92 (0.25) | 2.05 (0.31) | 2.01 (0.11) |

1) Mean ± Standard Deviation (N = 3).
2) Permeation rate of triplicate sample dosage units determined using 5-7 week old female hairless mouse skin in Chien et al. procedure for 146 hours and the rates determined from slope of Q vs permeation time plots.

Example 9

The procedure of Example 8 is followed to provide other transdermal dosage units of this invention: 1) 17-beta-estradiol in combination with norethindrone or norgestimate, 2) ethinyl estradiol-norgestimate

combination, and 3) the other combinations with progestins and estrogens selected from those named above, With the skin permeation rates necessary to provide the desired daily dose for fertility control or estradiol replacement. Also, the above dosage units of this Example and Example 8 are repeated using the other adhesives and polymers named above.

## Claims

1. A transdermal estrogen/progestin dosage unit for delivering estrogen/progestin hormones to a subject for fertility control or estrogen replacement therapy by absorption of said estrogen/progestin hormones through the skin of said subject, said unit comprising:
   a) a backing layer which is impervious to said estrogen and progestin hormones;
   b) a first layer which has been applied directly to said backing layer, and which is made of a polymer material having dissolved and/or microdispersed therein a dosage amount of one or more pharmaceutically acceptable estrogen hormones which are absorbable transdermally, said polymer material being bioacceptable, compatible with said estrogen hormones and permitting said estrogen hormones to be transmitted therethrough for transdermal absorption, and
   c) a second layer which is an adhesive and which has been applied directly to said first layer or optionally to a separating layer which has been applied directly to said first layer, and which is in intimate contact with said first layer, said second layer being made of a material different to the polymer material of the first layer or of a polymer material the same as that of the first layer, and having dissolved and/or microdispersed therein a dosage amount of one or more pharmaceutically acceptable progestin hormones which are absorbable transdermally, said second layer being bioacceptable, compatible with said progestin hormones, and permitting said progestin and estrogen hormones to be transmitted therethrough for transdermal absorption, said second layer comprising a transdermal skin absorption enhancing agent;

   said estrogen hormone being stable in said first and second layers and said progestin hormone being stable in said second layer, such that on application of said second layer of said dosage unit to the skin of a subject said estrogen and progestin hormones are transdermally absorbed simultaneously through the skin of the subject; and

   said dosage amounts of said estrogen hormones and said progestin hormones being such that said dosage unit is capable of delivering 20-50 mcg/day of 17-beta-estradiol (or an amount of estradiol or another estrogen having an equivalent estrogenic activity) and 100-1500 mcg/day of norgestimate or norethindrone (or an amount of another progestin having an equivalent progestin activity).

2. A dosage unit as claimed in Claim 1, which comprises further adhesion means in the form of a ring adhered to a region of said backing layer which extends beyond the sides of said first layer.

3. A dosage unit as claimed in Claim 1 or Claim 2 in which said estradiol is ethinyl estradiol or 17-beta-estradiol or combinations thereof.

4. A dosage unit as claimed in any one of Claims 1 to 3 in which said progestin is norethindrone or norgestimate or combinations thereof.

5. A dosage unit as claimed in Claim 1 in which said estrogen is ethinyl estradiol and said progestin is norethindrone or norgestimate.

6. A dosage unit as claimed in any one of the preceding claims wherein said second layer or said first layer or both layers are made from a polyacrylic adhesive polymer.

7. A dosage unit as claimed in any one of the preceding claims wherein the backing layer is microporous and breathable.

8. A dosage unit as claimed in Claim 1 wherein said second layer or first layer or both layers are made from a silicone adhesive polymer or a polyisobutylene adhesive polymer.

9. A dosage unit as claimed in any one of the preceding claims wherein the Enhancing Factor with regard to said progestin is at least 1.2, 1.3, 1.5 or 2.0.

**10.** A dosage unit as claimed in any one of the preceding claims wherein the skin permeation absorption enhancing agent is n-decyl alcohol.

**11.** A dosage unit as claimed in Claim 1 which provides at least minimum daily dosage amounts of said estrogen and progestin for about one week, having an amount of said skin permeation absorption enhancing agent to provide an Enhancing Factor of at least 1.5 with regard to said progestin.

**12.** A dosage unit as claimed in Claim 1 wherein said first layer and said second layer are separated by, but are in respective intimate contact with, a bioacceptable separating layer through which said one or more estrogens are transmissible, said separating layer being made of an adhesive or polymer which is free of estrogen, progestin and enhancing agents.

**13.** A dosage unit as claimed in Claim 12 in which said estrogen is ethinyl estradiol or 17-beta-estradiol or combinations thereof and said progestin is norethindrone or norgestimate or combinations thereof.

**14.** A dosage unit as claimed in Claim 13 or Claim 14 wherein the separating layer is made from a bioacceptable adhesive or polymer able to provide a dimensionally stable separating layer and a substantial reduction in the transmission rate of said estrogen.

**15.** A dosage unit as claimed in Claim 14 wherein the separating layer is made from a polyisobutylene adhesive.

**16.** A dosage unit as claimed in Claim 14 or Claim 15 wherein the ratio of transdermally absorbed progestin to estrogen hormones is in the range of 10/1 to 30/1.

**17.** A dosage unit as claimed in Claim 16 wherein the separating layer is made from polyisobutylene having a relative molecular mass $M_v$ (viscosity average) of from 800,000 to 900,000.

**18.** A fertility control kit comprising one or more series of three transdermal estrogen/progestin dosage units as claimed in Claim 1, and arranged to enable the carrying out of a process comprising:

a) applying to the skin of said subject a first dosage unit as claimed in Claim 1 which is capable of providing at least a minimum daily dosage of estrogen and progestin each day for approximately one week, continuously for approximately one week starting on about the fifth day of the menstrual cycle of said subject;

b) after such approximately one week period replacing said first dosage unit with a second such dosage unit and applying said second dosage unit for approximately one further week;

c) after said approximately one further week replacing said second dosage unit with a third such dosage unit and applying said third dosage unit for approximately one week, and repeating the sequence of steps a) to c) for each menstrual cycle as required so as to control said subject's fertility as required.

**Patentansprüche**

**1.** Transdermale Östrogen-/Progestin-Dosiereinheit zur Abgabe von Östrogen-/Progestinhormonan an eine Patientin zur Fertilitätskontrolle oder zur Östrogenersatztherapie durch Absorption der Östrogen-/Progestinhormone durch die Haut der Patientin, umfassend

a) eine Stützschicht, die für die Östrogen- und Progestinhormone undurchlässig ist;

b) eine erste Schicht, die direkt auf die Stützschicht aufgebracht ist, und die aus einem Polymermaterial hergestellt ist, in dem eine Dosismenge eines oder mehrerer pharmazeutisch verträglicher Östrogenhormone, die transdermal absorbierbar sind, aufgelöst und/oder mikrodispergiert ist, wobei das Polymermaterial biologisch verträglich, mit den Östrogenhormonen kompatibel ist und die Transmission der Östrogenhormone hindurch für die transdermale Absorption erlaubt, und

c) eine zweite Schicht, die adhäsiv ist und direkt auf die erste Schicht oder gegebenenfalls auf eine Trennschicht, die direkt auf die erste Schicht aufgebracht ist, aufgebracht ist, und die in innigem Kontakt mit der ersten Schicht ist, wobei die zweite Schicht aus einem Material, das sich von dem Polymermaterial der ersten Schicht unterscheidet, oder aus demselben Polymermaterial wie die erste Schicht hergestellt ist, und worin eine Dosismenge eines oder mehrerer pharmazeutisch verträglicher Progestinhormone, die transdermal absorbierbar sind, aufgelöst und/oder mikrodispergiert ist, wobei die zweite Schicht biologisch verträglich, mit den Progestinhormonen kompatibel ist und die Transmission der Progestin- und Östrogenhormone hindurch für die transdermale Absorption erlaubt, wobei die zweite Schicht ein Mittel zur Verstärkung der transdermalen Absorption durch die Haut umfaßt;

wobei das Östrogenhormon in der ersten und der zweiten Schicht stabil ist, und das Progestinhormon in der zweiten Schicht stabil ist, so daß bei Aufbringen der zweiten Schicht dar Dosiereinheit auf die Haut einer Patientin, die Östrogen- und Progestinhormone gleichzeitig durch die Haut der Patientin transdermal absorbiert werden; und wobei die Dosismengen der Östrogenhormone und dar Progestinhormone so sind, daß die Dosiereinheit 20-50 µg/Tag 17-β-Östradiol (oder eine Menge an Östradiol oder eines anderen Östrogens, das eine äquivalente Östrogenaktivität besitzt) und 100-1500 µg/Tag Norgestimat oder Norethindron (oder eine Menge einen anderen Progestins mit äquivalanter Progestinaktivität) abgeben kann.

**2.** Dosiereinheit nach Anspruch 1, umfassend weitere Adhäsionsmittel in Form eines Ringes, der in einem Bereich der Stützschicht haftet, der sich über die Seiten der ersten Schicht erstreckt.

**3.** Dosiereinheit nach Anspruch 1 oder 2, worin das Östradiol Ethinylöstradiol oder 17-β-Östradiol oder Kombinationen davon ist.

**4.** Dosiereinheit nach einem dar Ansprüche 1 bis 3, worin das Progestin Norethindron oder Norgestimat oder Kombinationen davon ist.

**5.** Dosiereinheit nach Anspruch 1, worin das Östrogen Ethinylöstradiol ist, und das Progestin Norethindron oder Norgestimat ist.

**6.** Dosiereinheit nach einem der vorausgehenden Ansprüche, worin die zweite Schicht oder die erste Schicht oder beide Schichten aus einem adhäsiven Polyacrylpolymeren hergestellt sind.

**7.** Dosiereinheit nach einem der vorausgehenden Ansprüche, worin die Stüztschicht mikroporös und atmungsaktiv ist.

**8.** Dosiereinheit nach Anspruch 1, worin die zweite Schicht oder die erste Schicht oder beide Schichten aus einem adhäsivan Silikonpolymeren oder einem adhäsiven Polyisobutylenpolymeren hergestellt sind.

**9.** Dosiereinheit nach einem der vorausgehenden Ansprüche, worin dar Verstärkungsfaktor bezüglich des Progestins mindestens 1,2, 1,3, 1,5 oder 2,0 beträgt.

**10.** Dosiereinheit nach einem dar vorausgehenden Ansprüche, worin das Mittel zur Verstärkung der Absorption durch die Haut n-Decylalkohol ist.

**11.** Dosiereinheit nach Anspruch 1, die mindestens minimale Tagesdosismengen an Östrogen und Progestin für etwa eine Woche liefert, wobei eine Menge an dem Mittel zur Verstärkung der Absorption durch die Haut vorhanden ist so daß ein Verstärkungsfaktor von mindestens 1,5 bezüglich des Progestins erzielt wird.

**12.** Dosiereinheit nach Anspruch 1, wobei die erste Schicht und die zweite Schicht durch eine biologisch verträgliche Trennschicht getrennt sind, aber in jeweiligem innigem Kontakt mit dieser sind, durch die ein oder mehrere Östrogen(e) dringen kann/können, wobei die Trennschicht aus einem Klebstoff oder einem Polymeren besteht, der/die frei von Östrogen, Progestin und Verstärkungsmittel ist.

**13.** Dosiereinheit nach Anspruch 12, worin das Östrogen Ethinylöstradiol oder 17-β-Östradiol oder Kombinationen davon ist, und das Progestin Norethindron oder Norgestimat oder Kombinationen davon ist.

**14.** Dosiereinheit nach Anspruch 13 oder 14, worin die Trennschicht aus einem biologisch verträglichen Klebstoff oder Polymeren besteht, dar/das eine dimensionsstabile Trennschicht und eine wesentliche Verringerung der Transmissionsrate das Östrogens ergeben kann.

**15.** Dosiereinheit nach Anspruch 14, worin die Trennschicht aus einem Polyisobutylenklebstoff hergestellt ist.

**16.** Dosiereinheit nach Anspruch 14 oder 15, worin das Verhältnis der transdermal absorbierten Progestin-: Östrogenhormone im Bereich von 10;1 bis 30:1 liegt.

**17.** Dosiereinheit nach Anspruch 16, worin die Trennschicht aus Polyisobutylen mit einer relativen Molekülmasse $M_v$ (Viskositätsdurchschnitt) von 800 000 bis 900 000 hergestellt ist.

**18.** Kit zur Kontrolle der Fertilität, umfassend eine oder mehrere Reihe(n) von drei transdermalen Östrogen/Progestin-Dosiereinheiten nach Anspruch 1, die so angeordnet sind, daß ein Verfahren durchgeführt werden kann, bei dem man
a) auf die Haut der Patientin eine erste Dosiereinheit nach Anspruch 1 aufbringt, die mindestens eine minimale Tagesdosis an Östrogen und Progestin jeden Tag für etwa eine Woche, kontinuierlich für etwa eine Woche beginnend etwa mit dem fünften Tag den Monatszyklus der Patientin abgeben kann;
b) nach etwa einer solchen Wochenperiode die erste Dosiereinheit durch eine zweite Dosiereinheit ersetzt und die zweite Dosiereinheit für etwa eine weitere Woche aufbringt;
c) nach etwa einer weiteren Woche die zweite Dosiereinheit durch eine dritte derartige Dosiereinheit ersetzt, und die dritte Dosiereinheit für etwa eine Woche aufbringt, und die Sequenzen der Stufen a) bis c) für jeden Monatszyklus, sooft notwendig, wiederholt, um die Fertilität der Patientin, wie benötigt, zu kontrollieren.

**Revendications**

**1.** Système d'administration transdermique d'oestrogène et de progestine pour l'administration des hormones oestrogène et progestine à un sujet à des fins de contrôle de la fertilité ou de thérapie de substitution en oestrogène par l'absorption desdites hormones oestrogène/progestine à travers la peau dudit sujet, ledit système comprenant :
a) une couche de soutien imperméable auxdites hormones oestrogène et progestine ;
b) une première couche directement appliquée sur ladite couche de soutien, constituée d'un matériau polymère dans lequel est dissous et/ou microdispersé une dose d'une ou plusieurs hormones oestrogènes pharmaceutiquement acceptables et absorbables par voie transdermique, ledit matériau polymère étant bioacceptable, compatible avec lesdites hormones oestrogènes et permettant auxdites hormones oestrogènes de le traverser pour une absorption transdermique, et
c) une seconde couche qui est un adhésif appliqué directement à ladite première couche ou éventuellement à une couche de séparation appliquée directement à ladite première couche, et qui est en contact étroit avec ladite première couche, ladite seconde couche étant composée d'un matériau polymère différent de celui de la première couche ou identique à celui-ci, et dans lequel est dissoute et/ou microdispersée une dose d'une ou plusieurs hormones progestines pharmaceutiquement acceptables, absorbables par voie transdermique, ladite seconde couche état bioacceptable, compatible avec lesdites hormones progestines et permettant auxdites hormones progestines et oestrogènes de la traverser pour une absorption transdermique, ladite seconde couche comprenant un agent facilitant l'absorption transdermique;

ladite hormone oestrogène étant stable dans lesdites première et deuxième couches et ladite hormone progestine étant stable dans ladite deuxième couche, de façon à ce que lors de l'application de ladite deuxième couche de ladite dose sur la peau d'un sujet, lesdites hormones oestrogène et progestine soient absorbées simultanément à travers la peau du sujet ; et lesdites doses desdites - hormones oestrogène et progestine étant telles que ladite dose est capable de délivrer 20-50 mcg/jour de 17-beta-oestradiol (ou une quantité d'oestradiol ou d'un autre oestrogène ayant une activité oestrogénique équivalente) et 100-1500 mcg/jour de noregestimate ou norethindrone (ou une quantité d'une autre progestine ayant une activité progestine équivalente).

**2.** Système selon la revendication 1 qui comprend des moyens d'adhésion complémentaires en forme d'anneau adhérant à la région de ladite couche de soutien qui s'étend au-delà des côtés de ladite première couche.

3. Système selon la revendication 1 ou 2, dans laquelle ledit oestradiol et l'étinyl oestradiol ou le 17-beta-oestradiol ou des combinaisons de ceux-ci.

4. Système selon l'une quelconque des revendications 1 à 3, dans laquelle ladite progestine et la norethindrone ou la norgestimate ou des combinaisons de celles-ci.

5. Système selon la revendication 1, dans laquelle ledit oestrogène est l'éthinyl oestradiol et ladite progestine est la norethindrone ou la norgestimate.

6. Système selon l'une quelconque des revendication précédentes, dans laquelle ladite seconde couche ou ladite première couche ou les deux couches sont composées d'un polymère adhésiv polyacrylique.

7. Système selon l'une quelconque des revendication précédentes, dans laquelle la couche de soutien est microporeuse et perméable à l'air.

8. Système selon la revendication 1, dans laquelle ladite seconde couche ou ladite première couche ou les deux couches sont composées d'un polymère de silicone adhésif ou d'un polymère de polyisobutylène adhésif.

9. Système selon l'une quelconque des revendication précédentes, dans laquelle le Facteur de Facilitation par rapport à ladite progestine est au moins 1.2, 1.3, 1.5, ou 2.0.

10. Système selon l'une quelconque des revendication précédentes, dans laquelle l'agent facilitant la perméation transdermique et le n-décyl alcool.

11. Système selon la revendication 1, qui fournit au moins des doses quotidiennes minimum desdites estrogène et progestine pour environ une semaine, comprenant une quantité dudit agent facilitant l'absorption par perméation de la peau pour fournir un Facteur de Facilitation d'au moins 1,5 par rapport à ladite progestine.

12. Système selon la revendication 1, dans laquelle ladite première couche et ladite seconde couche sont séparées par, mais sont en contact respectif intime avec, une couche de séparation bioacceptable à travers laquelle un ou plusieurs oestrogènes sont transmissibles, ladite couche séparatrice étant composée d'un adhésif ou d'un polymère libre d'oestrogène de progestine et d'agent facilitant.

13. Système selon la revendication 1, dans laquelle ledit oestrogène est l'éthinyl oestradiol ou le 17-beta-estradiol ou des combinaisons et ladite progestine est la norethindrone ou la norgestimate ou des combinaisons.

14. Système selon la revendication 13, dans laquelle la couche séparatrice est composée d'un adhésif bioacceptable ou d'un polymère capable de fournir une couche séparatrice stable au plan dimensionnel et une réduction substantielle dans la vitesse de transmission dudit oestrogène.

15. Système selon la revendication 14, dans laquelle la couche séparatrice est composée d'un adhésif polyisobutylène.

16. Système selon la revendication 14 ou 15, dans laquelle le rapport de progestine absorbée par voie transdermique aux hormones oestrogènes et dans une gamme de 10/1 à 30/1.

17. Système selon la revendication 16, dans laquelle la couche séparatrice est composée d'un polyisobutylène ayant une masse moléculaire relative $M_v$ (moyenne de viscosité) d'environ 800 000 à 900 000.

18. Kit de contrôle de la fertilité comprenant une ou plusieurs séries de trois systèmes d'administration transdermique oestrogène/progestine tels que revendiqués dans la revendication 1 et agencés de façon à permettre la mise en oeuvre d'une méthode comprenant:
a) l'application sur la peau dudit sujet d'un premier système tel que revendiqué dans la revendication 1 qui soit capable de fournir au moins une dose quotidienne minimale d'oestrogène et de progestine chaque jour pendant approximativement une semaine, de façon continue, pour approximativement une semaine commençant le 5ème jour du cycle menstruel dudit sujet ;
b) après une telle période d'environ une semaine, le remplacement dudit premier système par un second système et l'application dudit second système pendant approximativement une semaine suivante;
c) après ladite durée approximative d'une semaine, le remplacement dudit second système par un troisième système et l'application dudit troisième système pendant approximativement une semaine, et la répétition de la séquence des étapes a) à c) pour chaque cycle menstruel comme indiqué pour contrôler la fertilité dudit sujet selon la nécessité.

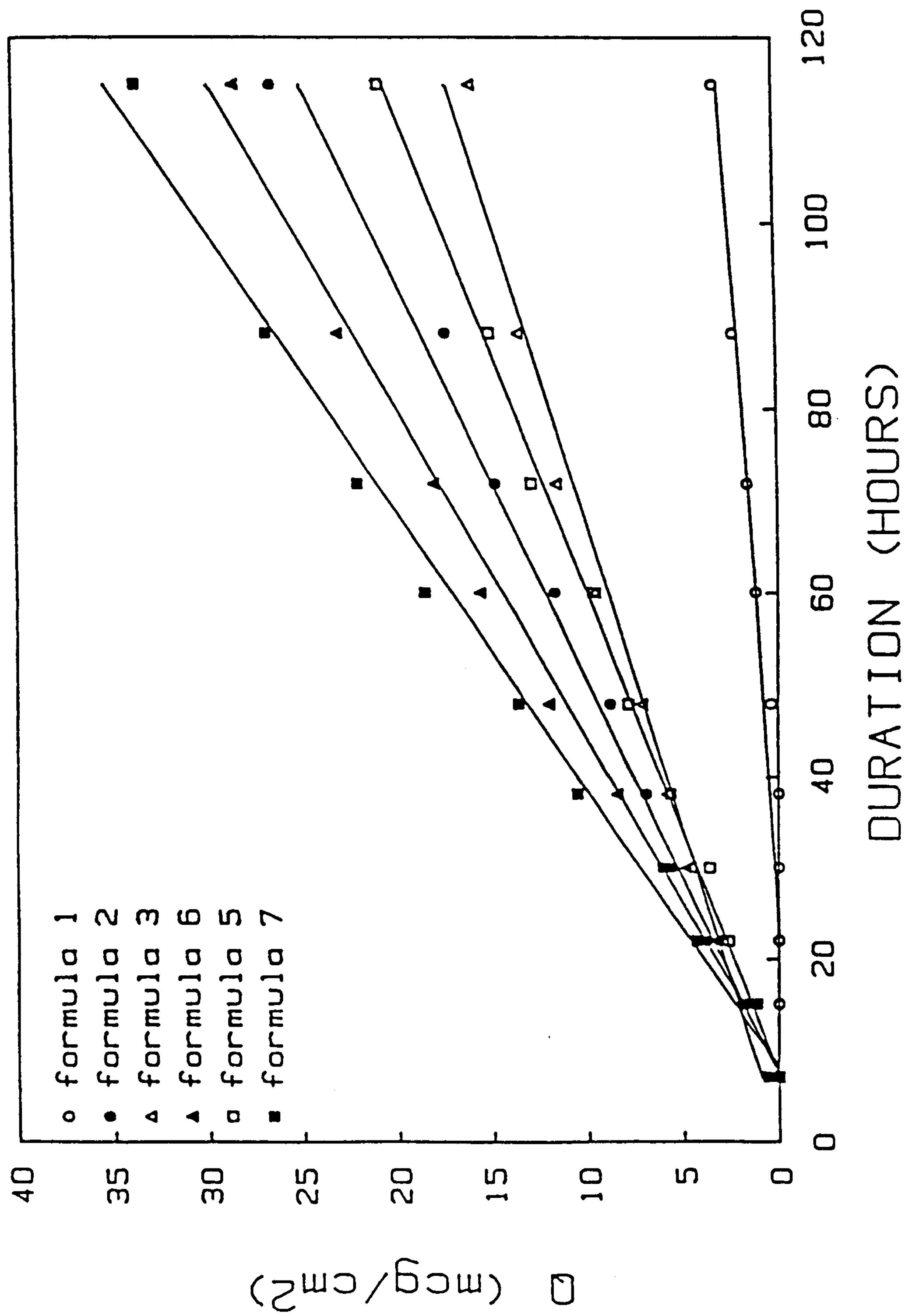
formula 1
formula 2
formula 3
formula 6
formula 5
formula 7
Q (mcg/cm2)
DURATION (HOURS)
0
5
10
15
20
25
30
35
40
0
20
40
60
80
100
120

FIG. I

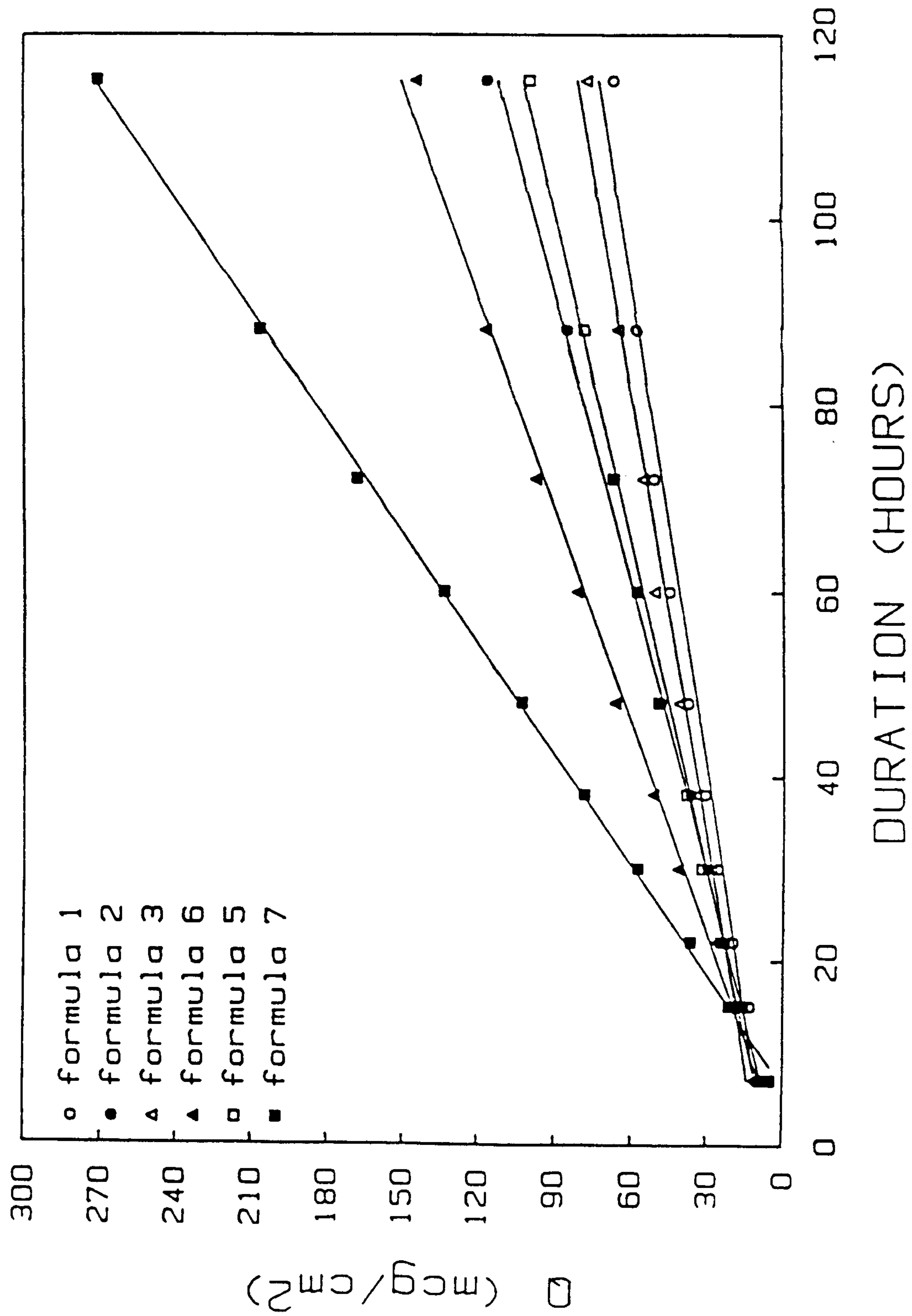

Q (mcg/cm2)
DURATION (HOURS)
formula 1
formula 2
formula 3
formula 6
formula 5
formula 7
0
30
60
90
120
150
180
210
240
270
300
0
20
40
60
80
100
120


FIG. 2

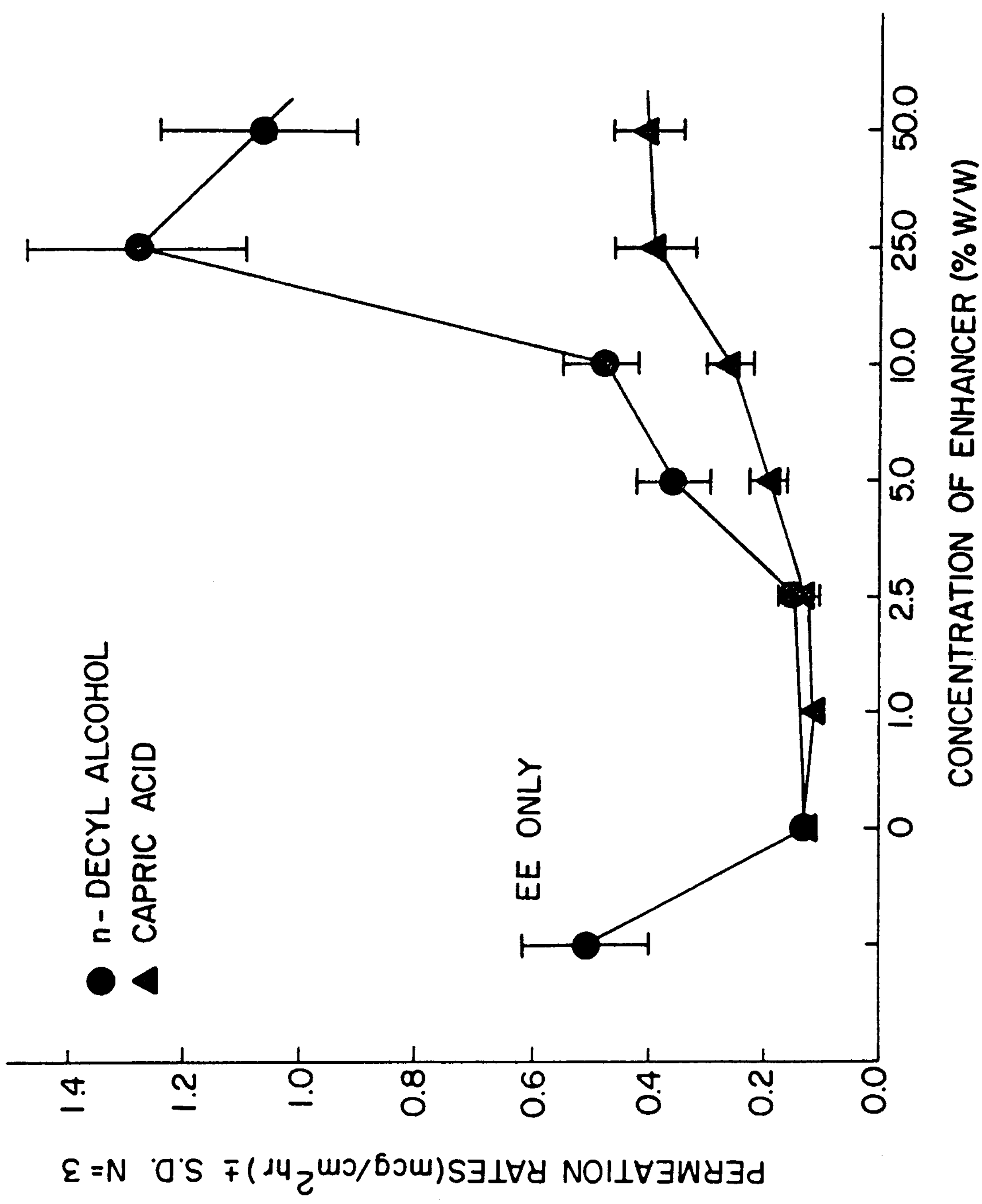

n- DECYL ALCOHOL
CAPRIC ACID
EE ONLY
PERMEATION RATES(mcg/cm2hr) ± S.D. N= 3
0.0
0.2
0.4
0.6
0.8
1.0
1.2
1.4
0
1.0
2.5
5.0
10.0
25.0
50.0
CONCENTRATION OF ENHANCER (% W/W)


FIG. 3

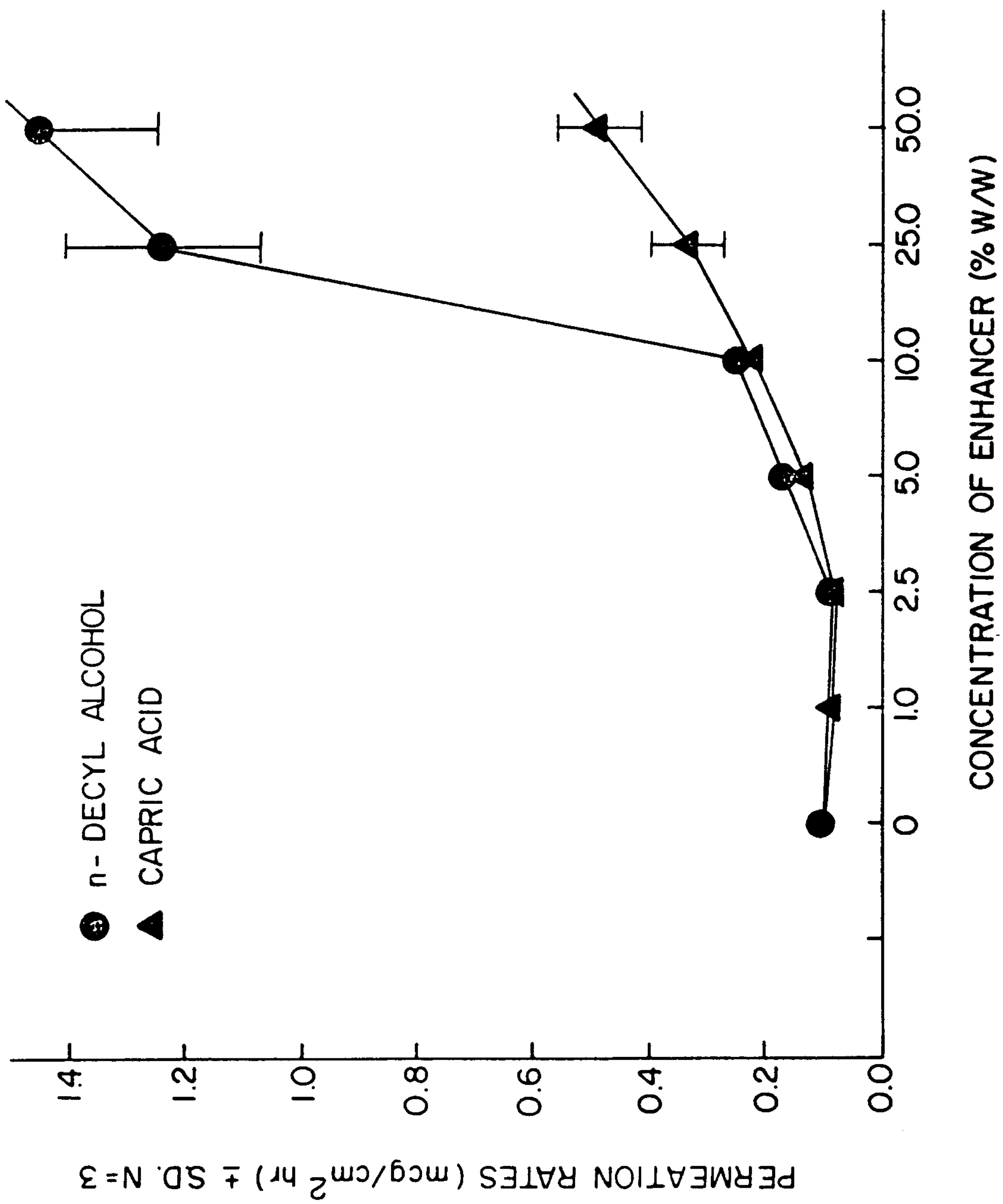
n - DECYL ALCOHOL
CAPRIC ACID
PERMEATION RATES (mcg/cm2 hr) ± S.D. N=3
1.4
1.2
1.0
0.8
0.6
0.4
0.2
0.0
0
1.0
2.5
5.0
10.0
25.0
50.0
CONCENTRATION OF ENHANCER (% W/W)

FIG. 4

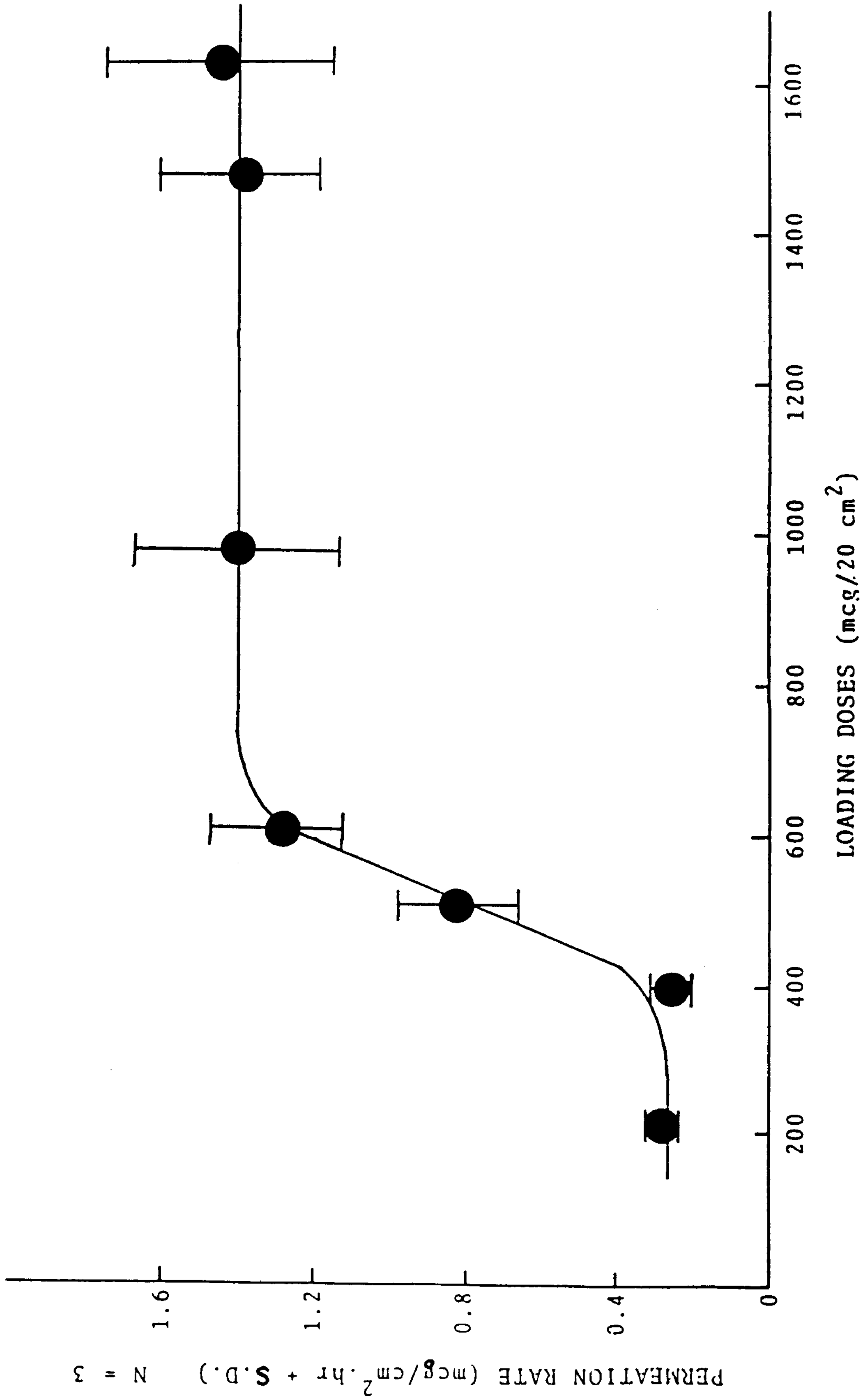

PERMEATION RATE (mcg/cm².hr + S.D.)
N = 3
0
0.4
0.8
1.2
1.6
200
400
600
800
1000
1200
1400
1600
LOADING DOSES (mcg/20 cm²)


FIG. 5